# EUROPÄISCHE PATENTSCHRIFT

(11) **EP 3 870 053 B1**
(45) Veröffentlichungstag und Bekanntmachung des Hinweises auf die Patenterteilung: **12.10.2022**
(21) Anmeldenummer: 19794511.6
(22) Anmeldetag: 23.10.2019
(51) Int. Cl.: A61B 6/00, A61B 6/04, G01N 23/04

(54) **PORTABLES SCHIENENSYSTEM ZUR HERSTELLUNG VON SCHNITTBILDAUFNAHMEN MITTELS EINER KONVENTIONELLEN RÖNTGENAUSRÜSTUNG**
PORTABLE RAIL SYSTEM FOR CREATING SECTIONAL IMAGE RECORDINGS BY MEANS OF CONVENTIONAL X-RAY EQUIPMENT
SYSTÈME DE RAIL PORTABLE POUR PRODUIRE DES IMAGES EN COUPE AU MOYEN D'UN ÉQUIPEMENT RADIOGRAPHIQUE CONVENTIONNEL

(30) Priorität: 25.10.2018 DE 102018126703; 07.03.2019 DE 202019101293 U
(43) Veröffentlichungstag der Anmeldung: 01.09.2021
(73) Patentinhaber: Universität Zürich, 8006 Zürich (CH)
(72) Erfinder: EPPENBERGER, Patrick, 8057 Zürich (CH)
(74) Vertreter: Schulz Junghans Patentanwälte PartGmbB
(86) Internationale Anmeldenummer: PCT/EP2019/078956
(87) Internationale Veröffentlichungsnummer: WO 2020/084023

(56) Entgegenhaltungen:
- CN-A- 1 392 402
- CN-A- 108 287 128
- US-A1- 2017 245 810

## Beschreibung

Die Erfindung betrifft ein Schienensystem mit dem eine Röntgenstrahlenquelle bezüglich eines Röntgenstrahlendetektors und eines abzubildenden Objekts angeordnet werden kann.

Mithilfe von Röntgenstrahlenquellen und entsprechenden Röntgenstrahlendetektoren werden herkömmliche Röntgenaufnahmen, d. h. Projektionsröntgenbilder, von Objekten erzeugt. In einem Projektionsröntgenbild werden mehrere Ebenen eines Objektes überlagert dargestellt. Herkömmliche Projektionsröntgenbilder werden für diagnostische medizinische Zwecke genutzt. Zudem finden herkömmliche Röntgenaufnahmen im Bereich der Forschung Anwendung beispielsweise in der Archäologie, der Bioarchäologie, der Anthropologie oder der Paläoanthropologie. Zudem können Röntgenaufnahmen in der Werkstoff- und Materialprüfung genutzt werden.

Bei einem Projektionsröntgenbild kann insbesondere bei einem Objekt, das eine komplexe innere Struktur aufweist, ein Artefakt, beispielsweise in Form einer Überlagerung, auftreten, so dass die Lesbarkeit der Aufnahme beeinträchtigt ist. Somit liefert eine herkömmliche Röntgenaufnahme in einem solchen Fall nur eine unvollständige Information über das Objekt, beispielsweise über die Anordnung einer ersten inneren Struktur bezüglich einer zweiten inneren Struktur.

Ferner sind tomographische Verfahren zur Bildgebung bekannt, beispielsweise die Computertomographie (CT), die mittels eines CT-Gerätes durchgeführt werden kann. Dabei werden Projektionsröntgenbilder aus einer Vielzahl unterschiedlicher Richtungen aufgenommen und aus diesen Projektionsröntgenbildern Röntgenschnittbilder berechnet bzw. rekonstruiert, die einzelne Ebenen des Objektes wiedergeben. Mithilfe von Röntgenschnittbildern kann eine innere Struktur eines untersuchten Objektes dargestellt werden, bzw. die Anordnung mehrerer innerer Strukturen zueinander. Eine komplexe Struktur, die bei einer herkömmlichen Röntgenaufnahme aufgrund eines Überlagerungsartefaktes nicht darstellbar sein kann, kann mithilfe eines Röntgenschnittbildes dargestellt werden, so dass eine derartige Darstellung mehr Informationen über das untersuchte Objekt bereitstellen kann als ein Projektionsröntgenbild.

Neben CT-Geräten sind sogenannte Mikro-CT-Geräte bekannt, mit denen eine hochauflösende Tomographie durchgeführt werden kann.

Dokument US 2017/245810 A1 offenbart einen System zum Anordnen einer Röntgenstrahlenquelle bezüglich eines Röntgenstrahlendetektors und eines abzubildenden Objekts mit:
- einem Arm, die dazu ausgebildet ist, eine Röntgenstrahlquelle zu tragen,
- einem Träger, die dazu ausgebildet ist, einen Röntgenstrahlendetektor zu tragen,
- einer wobei der Arm und Träger dazu ausgebildet sind, zum Positionieren der Röntgenstrahlenquelle bezüglich des Röntgenstrahlendetektors zueinander verschiebbar auf der Führungsschiene angeordnet zu werden, wobei der Arm und Träger lösbar an der Führungsschiene festlegbar sind, um eine Position der Röntgenstrahlenquelle bezüglich des Röntgenstrahlendetektors zu fixieren, und
- einem Träger, der zum Tragen des mittels Röntgenstrahlung der Röntgenstrahlenquelle abzubildenden Objekts ausgebildet ist, wobei der Träger weiterhin zum Positionieren des Trägers zwischen der Röntgenstrahlenquelle und dem Röntgenstrahlendetektor dazu ausgebildet ist, verschiebbar auf dem Arm angeordnet zu werden, wobei der Träger an der jeweiligen Gleitschiene indirekt festlegbar ist, um eine Position des Trägers bezüglich der Röntgenstrahlenquelle und des Röntgenstrahlendetektors zu fixieren.

CT-Geräte und Mikro-CT-Geräte sind für einen stationären Gebrauch vorgesehen, da sie nicht einfach auf- oder abbaubar sind. Zudem sind die Anschaffungskosten eines derartigen Gerätes hoch.

Daher ist eine Vorrichtung von Interesse, mit der auf einfache und kostengünstige Weise Projektionsröntgenbilder aus unterschiedlichen Richtungen aufgenommen und aus ihnen Schnittbilder rekonstruiert werden können, wobei die Vorrichtung einfach zusammen- und auseinanderbaubar ist, so dass die Vorrichtung einfach transportiert werden kann, beispielsweise an unterschiedliche Einsatzorte.

Diese Aufgabe wird durch das Schienensystem nach Anspruch 1 oder die bildgebende Einrichtung nach Anspruch 21 bzw. das Verfahren nach Anspruch 25 gelöst.

Ein erster Aspekt der Erfindung betrifft ein Schienensystem zum Anordnen einer Röntgenstrahlenquelle bezüglich eines Röntgenstrahlendetektors und eines abzubildenden Objektes gemäß Anspruch 1.

Das Schienensystem kann eine erste Gleitschiene aufweisen. Die erste Gleitschiene kann dazu ausgebildet sein, eine Röntgenstrahlquelle zu tragen. Zudem kann das Schienensystem eine zweite Gleitschiene aufweisen, wobei die zweite Gleitschiene dazu ausgebildet sein kann, einen Röntgenstrahlendetektor zu tragen. Ferner kann das Schienensystem eine Führungsschiene, die zum Führen der ersten und der zweiten Gleitschiene ausgebildet ist, aufweisen, wobei die erste und die zweite Gleitschiene jeweils dazu ausgebildet sind, zum Positionieren der Röntgenstrahlenquelle bezüglich des Röntgenstrahlendetektors zueinander verschiebbar auf der Führungsschiene angeordnet zu werden, wobei die erste und die zweite Gleitschiene lösbar an der Führungsschiene festlegbar sind, um eine Position der Röntgenstrahlenquelle bezüglich des Röntgenstrahlendetektors zu fixieren. Das Schienensystem kann einen Träger aufweisen, der zum Tragen des mittels Röntgenstrahlung der Röntgenstrahlenquelle abzubildenden Objekts ausgebildet ist. Der Träger kann weiterhin zum Positionieren des Trägers zwischen der Röntgenstrahlenquelle und dem Röntgenstrahlendetektor ausgebildet sein, wobei der Träger dazu ausgebildet ist, verschiebbar auf der ersten Gleitschiene oder der zweiten Gleitschiene angeordnet zu werden, wobei der Träger an der jeweiligen Gleitschiene festlegbar ist, um eine Position des Trägers bezüglich der Röntgenstrahlenquelle und des Röntgenstrahlendetektors zu fixieren.

Der Träger kann eine Halterung aufweisen, mit der das Objekt am Träger angeordnet werden kann. In einer Ausführungsform kann der Träger eine Plattform aufweisen, auf der das Objekt positioniert werden kann.

Durch das Schienensystem kann auf einfache Weise die Position zwischen der Röntgenstrahlenquelle, dem Röntgenstrahlendetektor und einem auf dem Träger angeordneten Objekt eingestellt und konstant gehalten werden.

In einer Ausführungsform kann sich die Führungsschiene entlang einer Längsrichtung erstrecken. Die erste und die zweite Gleitschiene können entlang der Längsrichtung bezüglich der Führungsschiene bewegbar sein, so dass eine Position der Röntgenstrahlenquelle bezüglich des Röntgenstrahlendetektors einstellbar ist.

Insbesondere kann der Abstand der ersten und der zweiten Gleitschiene zueinander auf einfache Weise verändert werden, so dass auch ein Abstand zwischen der Röntgenstrahlenquelle (die auf der ersten Gleitschiene angeordnet sein kann) und dem Röntgenstrahlendetektor (der auf der zweiten Gleitschiene angeordnet sein kann) verändert und genau eingestellt werden kann, ohne dass die Röntgenstrahlenquelle und/oder der Röntgenstrahlendetektor abmontiert, ausgerichtet und neu montiert werden müssen.

In einer Ausführungsform ist das Schienensystem dadurch charakterisiert, dass der Träger bezüglich der ersten oder der zweiten Gleitschiene um eine Drehachse drehbar ist. Dabei kann sich die Drehachse orthogonal zur Längsrichtung der Führungsschiene erstrecken. Ein auf dem Träger angeordnetes Objekt kann um einen definierten Drehwinkel aus einer ersten Position in eine zweite Position drehbar sein. In einer erfindungsgemäßen Ausführungsform kann ein auf dem Träger angeordnetes Objekt aus einer Anfangsposition sukzessive in einer Vielzahl Drehschritten um jeweils einen definierten Drehwinkel drehbar sein, so dass nach einer definierten Anzahl an Drehschritten das Objekt wieder in der Anfangsposition ist.

Wenn eine Röntgenstrahlenquelle und ein Röntgenstrahlendetektor am Schienensystem angeordnet sind und das Objekt zwischen der Röntgenstrahlenquelle und dem Röntgenstrahlendetektor angeordnet ist, kann ein erstes Projektionsröntgenbild des Objektes in der ersten Position und ein zweites Projektionsröntgenbild des Objektes in der zweiten Position erzeugt werden. Das bedeutet, dass durch das Drehen des Trägers auf einfache Weise Projektionsröntgenbilder des Objektes aus unterschiedlichen Richtungen erzeugt werden können. Durch die Projektionsröntgenbilder aus unterschiedlichen Richtungen können mehr Informationen über das Objekt erlangt werden als durch ein Projektionsröntgenbild aus nur einer Richtung. Sie können zudem die Grundlage sein für das Erstellen eines Röntgenschnittbildes. Weiterhin können Projektionsröntgenbilder aus unterschiedlichen Richtungen genutzt werden für eine Berechnung einer dreidimensionalen Rekonstruktion des Objektes.

Das Objekt kann nach einer Vielzahl an Drehschritten einmal um sich selbst gedreht sein, d. h. eine 360°-Drehung durchgeführt haben. Der vordefinierte Drehwinkel kann insbesondere ein Winkel sein, der ein Teiler des Vollwinkels ist.

Je kleiner der vordefinierte Drehwinkel ist, d. h. je mehr Projektionsröntgenbilder des Objektes erzeugt werden, desto genauer kann das Objekt dargestellt werden.

Wenn eine Röntgenstrahlenquelle und ein Röntgenstrahlendetektor am Schienensystem angeordnet sind und das Objekt zwischen der Röntgenstrahlenquelle und dem Röntgenstrahlendetektor angeordnet ist, kann eine Vielzahl an Projektionsröntgenbildern des Objektes aus einer Vielzahl an Richtungen erzeugt werden.

Mithilfe des Schienensystems kann eine Distanz zwischen der Röntgenstrahlenquelle und der Drehachse genau eingestellt werden. Bei einer wiederholten Untersuchung eines Objektes kann die gleiche Distanz erneut eingestellt werden.

Das Schienensystem kann einen Schrittmotor aufweisen, der dazu ausgebildet ist, den Träger um die Drehachse zu drehen.

Mithilfe des Schrittmotors kann der Träger automatisch und exakt um einen vordefinierten Drehwinkel gedreht werden. Der Schrittmotor kann so eingerichtet sein, dass eine gewisse Wartezeit zwischen zwei aufeinanderfolgenden Drehschritten eingehalten wird. In dieser Wartezeit kann beispielsweise ein Projektionsröntgenbild des Objektes in der jeweiligen Position erzeugt werden.

In einer erfindungsgemäßen Ausführungsform kann das Schienensystem eine Steuervorrichtung aufweisen, die dazu ausgebildet ist, den Schrittmotor anzusteuern.

Die Steuervorrichtung kann beispielsweise eine Computersteuerung sein.

Mithilfe einer Steuervorrichtung kann der Schrittmotor angesteuert werden. Auf einfache Weise kann beispielsweise der vordefinierte Drehwinkel für unterschiedliche Untersuchungen verändert werden.

Eine Ausführungsform der Erfindung weist einen Aktuator auf, der dazu ausgebildet ist, den Träger entlang der Längsrichtung zu bewegen.

Der Träger kann schrittweise in Richtung des Röntgenstrahlendetektors bewegt werden. Der Aktuator kann alternativ dazu ausgebildet sein, den Träger in Richtung der Röntgenstrahlenquelle zu bewegen.

In einer Ausführungsform des Schienensystems kann der Träger entlang der Längsrichtung bewegt und gedreht werden. Gemäß einer Ausführungsform kann der Träger derart bewegt werden, dass gleichzeitig eine Drehbewegung und eine Bewegung entlang der Längsrichtung ausgeübt werden kann. Alternativ kann eine Drehbewegung der Bewegung entlang der Längsrichtung vorangehen oder nachfolgen.

Gemäß einer erfindungsgemäßen Ausführungsform kann die Führungsschiene eine erste Seitenfläche und eine von der ersten Seitenfläche abgewandte zweite Seitenfläche aufweisen. Die erste Seitenfläche kann eine erste Nut und die zweite Seitenfläche kann eine zweite Nut aufweisen. Die erste und die zweite Nut können sich entlang der Längsrichtung erstrecken. Ferner kann die erste Gleitschiene eine erste Bodenfläche aufweisen, wobei die erste Bodenfläche eine dritte und eine vierte Nut aufweisen kann. Die zweite Gleitschiene kann eine zweite Bodenfläche aufweisen, wobei die zweite Bodenfläche eine fünfte Nut und eine sechste Nut aufweisen kann. Die dritte, vierte, fünfte und sechste Nut können sich entlang der Längsrichtung erstrecken. Das Schienensystem kann zudem zumindest ein erstes Tragelement, ein zweites Tragelement, ein drittes Tragelement und ein viertes Tragelement aufweisen. Weiterhin kann das Schienensystem zumindest einen ersten Nutenstein, einen zweiten Nutenstein, einen dritten Nutenstein und einen vierten Nutenstein aufweisen, wobei der erste Nutenstein in der dritten Nut, der zweite Nutenstein in der vierten Nut, der dritte Nutenstein in der fünften Nut und der vierte Nutenstein in der sechsten Nut anordenbar sind. Dabei sind der erste Nutenstein am ersten Tragelement, der zweite Nutenstein am zweiten Tragelement, der dritte Nutenstein am dritten Tragelement und der vierte Nutenstein am vierten Tragelement festlegbar. Ferner ist ein erstes Klemmelement am ersten Tragelement, ein zweites Klemmelement am zweiten Tragelement, ein drittes Klemmelement am dritten Tragelement und ein viertes Klemmelement am vierten Tragelement angeordnet. Das erste und das dritte Klemmelement können in der ersten Nut positionierbar sein und das zweite und das vierte Klemmelement können in der zweiten Nut positionierbar sein, so dass die erste Gleitschiene und die zweite Gleitschiene mit der Führungsschiene verbindbar sein können.

Das bedeutet, dass ein Tragelement sowohl mit der Führungsschiene als auch mit der ersten oder der zweiten Gleitschiene verbunden werden kann. Mithilfe eines entsprechenden Tragelementes kann die erste oder die zweite Gleitschiene an der Führungsschiene positioniert werden.

Die erste Gleitschiene kann unter Verwendung des ersten und des zweiten Tragelementes an der Führungsschiene angeordnet werden. Die zweite Gleitschiene kann mithilfe des dritten und des vierten Tragelementes an der Führungsschiene angeordnet werden.

In einer Ausführungsform kann das erste Klemmelement in der ersten Nut verklemmbar sein, so dass das erste Tragelement an der Führungsschiene fixierbar ist. Das zweite Klemmelement kann in der zweiten Nut verklemmbar sein, so dass das zweite Tragelement an der Führungsschiene fixierbar ist. Das dritte Klemmelement kann in der ersten Nut verklemmbar sein, so dass das dritte Tragelement an der Führungsschiene fixierbar ist. Ferner kann das vierte Klemmelement in der zweiten Nut verklemmbar sein, so dass das vierte Tragelement an der Führungsschiene fixierbar ist.

Das erste Klemmelement kann derart ausgebildet und angeordnet sein, dass es zumindest einen ersten und einen zweiten Zustand annehmen kann, wobei das erste Klemmelement im ersten Zustand in der ersten Nut verklemmt ist und das erste Klemmelement im zweiten Zustand in der ersten Nut bewegbar, d. h. nicht verklemmt ist. In einer Ausführungsform kann das erste Klemmelement einfach vom ersten Zustand in den zweiten Zustand und vom zweiten Zustand in den ersten Zustand überführt werden. Mit anderen Worten kann das erste Klemmelement einfach verklemmt und gelöst werden.

In einer Ausführungsform sind das zweite, das dritte und/oder das vierte Klemmelement analog zum ersten Klemmelement ausgebildet.

Durch Verklemmen des ersten und zweiten Klemmelementes kann die erste Gleitschiene an der Führungsschiene fixiert werden. Werden das erste und das zweite Klemmelement in den zweiten Zustand überführt, wird die Fixierung der ersten Gleitschiene an der Führungsschiene gelöst und die erste Gleitschiene kann beispielsweise einfach entlang der Führungsschiene verschoben werden. Anschließend können das erste und das zweite Klemmelement wieder verklemmt werden, so dass die erste Gleitschiene erneut an der Führungsschiene festgelegt sein kann.

Entsprechend kann die zweite Gleitschiene mithilfe des dritten und des vierten Klemmelementes an der Führungsschiene fixiert werden, wobei die zweite Gleitschiene entlang der Führungsschiene verschoben werden kann, wenn das dritte und das vierte Klemmelement gelöst worden sind, d. h. im zweiten Zustand vorliegen.

Gemäß einer Ausführungsform kann das erste Tragelement eine erste Tragfläche und eine erste Anlagefläche aufweisen, wobei die erste Tragfläche und die erste Anlagefläche orthogonal zueinander angeordnet sind. Das zweite Tragelement kann eine zweite Tragfläche und eine zweite Anlagefläche aufweisen, die orthogonal zueinander angeordnet sind. Das dritte Tragelement kann eine dritte Tragfläche und eine dritte Anlagefläche aufweisen, die orthogonal zueinander angeordnet sind. Das vierte Tragelement kann eine vierte Tragfläche und eine vierte Anlagefläche aufweisen, wobei die vierte Tragfläche und die vierte Anlagefläche orthogonal zueinander angeordnet sind.

In einer Ausführungsform kann das erste Tragelement derart in Bezug zur Führungsschiene und zur ersten Gleitschiene positionierbar sein, dass die erste Tragfläche an der ersten Bodenfläche anliegt und die erste Anlagefläche an der ersten Seitenfläche anliegt. Das zweite Tragelement kann derart in Bezug zur Führungsschiene und zur ersten Gleitschiene positionierbar sein, dass die zweite Tragfläche an der ersten Bodenfläche anliegt und die zweite Anlagefläche an der zweiten Seitenfläche anliegt.

Das erste und das zweite Tragelement können derart an der Führungsschiene positioniert sein, dass sie einander gegenüberliegen. Die erste Gleitschiene kann sowohl an dem ersten als auch an dem zweiten Tragelement anliegen, insbesondere kann die erste Bodenfläche an der ersten und der zweiten Tragfläche anliegen.

Das dritte Tragelement kann derart in Bezug zur Führungsschiene und zur zweiten Gleitschiene positionierbar sein, dass die dritte Tragfläche an der zweiten Bodenfläche anliegt und die dritte Anlagefläche an der ersten Seitenfläche anliegt. Das vierte Tragelement kann derart in Bezug zur Führungsschiene und zur zweiten Gleitschiene positionierbar sein, dass die vierte Tragfläche an der zweiten Bodenfläche anliegt und die vierte Anlagefläche an der zweiten Seitenfläche anliegt.

Das bedeutet, dass das dritte und das vierte Tragelement derart an der Führungsschiene positioniert sein können, dass sie einander gegenüberliegen. Die zweite Gleitschiene kann sowohl an dem dritten als auch an dem vierten Tragelement anliegen.

Gemäß einer Ausführungsform kann das Schienensystem ein erstes Befestigungsmittel aufweisen, das dazu ausgebildet ist, den ersten Nutenstein in der dritten Nut festzulegen. Ferner kann das Schienensystem ein zweites Befestigungsmittel aufweisen, das dazu ausgebildet ist, den zweiten Nutenstein in der vierten Nut festzulegen. Das Schienensystem kann ein drittes Befestigungsmittels aufweisen, das dazu ausgebildet ist, den dritten Nutenstein in der fünften Nut festzulegen. In einer Ausführungsform kann das Schienensystem ein viertes Befestigungsmittel aufweisen, das dazu ausgebildet ist, den vierten Nutenstein in der sechsten Nut festzulegen.

Das erste, zweite, dritte und/oder vierte Befestigungsmittel kann beispielsweise jeweils eine Schraube sein.

In einer erfindungsgemäßen Ausführungsform können die Führungsschiene, die erste Gleitschiene und die zweite Gleitschiene dazu ausgebildet und derart zueinander angeordnet sein, dass die erste und die zweite Seitenfläche der Führungsschiene senkrecht zur ersten Bodenfläche verlaufen und die erste und die zweite Seitenfläche senkrecht zur zweiten Bodenfläche verlaufen.

In einer Ausführungsform kann der Träger einen Trägernutenstein aufweisen. Ferner kann die erste Gleitschiene eine erste Deckfläche aufweisen, wobei die erste Deckfläche eine erste Trägernut aufweisen und sich entlang der Längsrichtung erstrecken kann. Der Trägernutenstein kann dazu ausgebildet sein, in der ersten Trägernut angeordnet zu werden. Gemäß der Erfindung kann die zweite Gleitschiene eine zweite Deckfläche aufweisen, wobei die zweite Deckfläche eine zweite Trägernut aufweist. Die zweite Trägernut kann sich entlang der Längsrichtung erstrecken, und der Trägernutenstein kann dazu ausgebildet sein, in der zweiten Trägernut angeordnet zu werden.

Der Trägernutenstein kann mithilfe eines Befestigungsmittels, beispielsweise einer Schraube, lösbar in der ersten Trägernut oder der zweiten Trägernut befestigt werden.

Mithilfe des Trägernutensteins kann der Träger einfach an der ersten Gleitschiene oder der zweiten Gleitschiene angeordnet werden. Ein Abstand zwischen dem Träger und der Röntgenstrahlenquelle und/oder ein Abstand zwischen dem Träger und dem Röntgenstrahlendetektor kann leicht verändert werden, indem der Trägernutenstein gelöst, verschoben und erneut fixiert wird.

Erfindungsgemäß kann der Träger ein Gelenk aufweisen, so dass der Träger um eine Kippachse kippbar ist, wobei die Kippachse orthogonal zur Längsrichtung der Führungsschiene verläuft.

In einer Ausführungsform weist das Schienensystem eine Mehrzahl an Stützelementen auf, wobei das jeweilige Stützelement ein Winkelstück, eine am Winkelstück anordenbare Verbindungsstange und einen an der Verbindungsstange angeordneten Fuß aufweist. Das Winkelstück kann dazu ausgebildet sein, an der Führungsschiene festgelegt zu werden. Mithilfe der Verbindungsstange kann ein Abstand zwischen dem Winkelstück und dem Fuß einstellbar sein.

Die Verbindungsstange kann eine Gewindestange sein.

Die Stützelemente können dazu ausgebildet sein und die Abstände zwischen dem Fuß und dem Winkelstück derart eingestellt sein, dass die Längsrichtung der Führungsschiene eine vorgegebene Richtung einnimmt. Beispielsweise kann mithilfe der Stützelemente die Führungsschiene so ausgerichtet werden, dass die Längsrichtung waagerecht ausgerichtet ist. Mittels der Stützelemente können beispielsweise Unebenheiten im Boden ausgeglichen werden. Insbesondere bei einer Verwendung des Schienensystems im Freien, beispielsweise bei archäologischen Ausgrabungen, ist dies von Vorteil, da keine zusätzlichen Kosten entstehen, um einen geeigneten Untergrund für das Schienensystem bereitzustellen.

Alternativ kann mithilfe der Stützelemente das Schienensystem gekippt werden, so dass beispielsweise die Röntgenstrahlenquelle oberhalb von dem Röntgenstrahlendetektor positioniert ist. Das dazwischen angeordnete Objekt kann beispielsweise auf einem Träger angeordnet sein, der ein Gelenk aufweist, so dass das Objekt ebenfalls gekippt werden kann. In einer Ausführungsform kann das Schienensystem so ausgebildet sein, dass die Röntgenstrahlenquelle oberhalb von dem Röntgenstrahlendetektor angeordnet ist, wobei der Träger derart gekippt ist, dass das Objekt waagerecht ausgerichtet ist.

Erfindungsgemäß kann das Winkelstück Magnesium aufweisen oder aus einer Metalllegierung ausgebildet sein, die Magnesium aufweist.

In einer Ausführungsform können die Führungsschiene, die erste Gleitschiene und die zweite Gleitschiene Aluminium aufweisen oder aus einer Aluminiumlegierung ausgebildet sein.

Aluminium ist ein leichtes Material, so dass in dieser Ausführungsform das Schienensystem ein geringes Gewicht hat. Auch Magnesium besitzt ein geringes Gewicht.

Durch das geringe Gewicht ist ein Transport des Schienensystems einfach. Auch der Aufbau und/oder der Abbau des Schienensystems werden bzw. wird durch das geringe Gewicht erleichtert, da wenig Kraft benötigt wird. In einer Ausführungsform kann das Schienensystem von einer einzelnen Person getragen werden. Ferner kann das Schienensystem von einer einzelnen Person montiert, d. h. aufgebaut und/oder abgebaut, werden.

Gemäß einer Ausführungsform können die erste Gleitschiene, die zweite Gleitschiene und der Träger lösbar an der Führungsschiene festlegbar sein, so dass das Schienensystem in die erste Gleitschiene, die zweite Gleitschiene, den Träger und die Führungsschiene zerlegbar ist.

Das bedeutet, dass die erste und die zweite Gleitschiene von der Führungsschiene entfernt werden können. Das Schienensystem kann in die einzelnen Komponenten zerlegt werden. Das erleichtert einen Transport des Schienensystems. Das Schienensystem kann anschließend erneut zusammengebaut werden.

In einer Ausführungsform kann das Schienensystem eine Analyseeinrichtung aufweisen, wobei die Analyseeinrichtung zur Bearbeitung, Auswertung und/oder Speicherung eines Datensatzes ausgebildet ist.

Ein Datensatz kann beispielsweise eine Vielzahl von Datenpunkten eines Projektionsröntgenbildes aufweisen.

Nach einer Ausführungsform kann die Analyseeinrichtung dazu ausgebildet sein, einen Transformationsalgorithmus auszuführen, der anhand einer Vielzahl von Projektionsröntgenbildern mindestens ein Röntgenschnittbild des Objektes erstellt.

In einer Ausführungsform kann anhand der Vielzahl von Projektionsröntgenbildern eine dreidimensionale Rekonstruktion des Objektes erstellt werden.

Erfindungsgemäß kann der Transformationsalgorithmus ein Kegelstrahlalgorithmus sein.

Ein weiterer Aspekt der Erfindung betrifft eine bildgebende Einrichtung zum Erzeugen von Röntgenschnittbildern eines Objekts gemäß Anspruch 21.

Die bildgebende Einrichtung weist ein erfindungsgemäßes Schienensystem auf, sowie ferner eine Röntgenstrahlenquelle und einen Röntgenstrahlendetektor, wobei die Röntgenstrahlenquelle an der ersten Gleitschiene festgelegt ist, und wobei der Röntgenstrahlendetektor an der zweiten Gleitschiene festgelegt ist.

Gemäß einer Ausführungsform kann der Röntgenstrahlendetektor eine digitale Röntgenstrahlendetektorplatte aufweisen.

Mittels einer digitalen Röntgenstrahlendetektorplatte können Projektionsröntgenbilder digital aufgenommen werden. Die von der digitalen Röntgenstrahlendetektorplatte aufgenommenen Daten können an eine Analyseeinrichtung, z. B. einen Computer, weitergegeben und bearbeitet, ausgewertet und/oder gespeichert werden.

Der Röntgenstrahlendetektor kann ein portabler Röntgenstrahlendetektor sein.

Nach einer Ausführungsform kann die Röntgenstrahlenquelle eine portable Röntgenstrahlenquelle sein.

In einer Ausführungsform können die Röntgenstrahlenquelle eine portable Röntgenstrahlenquelle und der Röntgenstrahlendetektor ein portabler Röntgenstrahlendetektor sein.

Eine Ausführungsform ist dadurch gekennzeichnet, dass die bildgebende Einrichtung eine Analyseeinrichtung aufweist, wobei die Analyseeinrichtung dazu ausgebildet sein kann, einen Transformationsalgorithmus auszuführen, der anhand einer Vielzahl von Röntgenprojektionsbildern mindestens ein Röntgenschnittbild des Objektes erstellt.

Gemäß der Erfindung kann die Analyseeinrichtung dazu ausgebildet sein, eine dreidimensionale Rekonstruktion des Objektes anhand einer Vielzahl von Röntgenprojektionsbildern zu erstellen.

Die Analyseeinrichtung kann eine Software aufweisen, die dazu ausgebildet ist, mindestens ein Röntgenschnittbild des Objektes zu erstellen und/oder die dreidimensionale Rekonstruktion des Objektes zu erzeugen.

Ein weiterer Aspekt der Erfindung betrifft ein Verfahren zum Erzeugen eines Röntgenprojektionsbildes eines Objekts unter Verwendung eines erfindungsgemäßen Schienensystems oder unter Verwendung einer erfindungsgemäßen bildgebenden Einrichtung, wobei ein Objekt auf dem Träger des Schienensystems angeordnet wird und mittels der an der ersten Gleitschiene festgelegten Röntgenstrahlenquelle und des an der zweiten Gleitschiene festgelegten Röntgenstrahlendetektors zumindest ein Projektionsröntgenbild des Objekts erzeugt wird.

Gemäß einer Ausführungsform kann das auf dem Träger angeordnete Objekt mittels des erfindungsgemäßen Schienensystems aus einer ersten Position in eine zweite Position gedreht werden und ein erstes Projektionsröntgenbild des Objektes in der ersten Position und ein zweites Projektionsröntgenbild des Objektes in der zweiten Position erzeugt werden.

In einer Ausführungsform kann anhand einer Vielzahl an Projektionsröntgenbildern mindestens ein Röntgenschnittbild des Objektes erstellt werden. In einer Ausführungsform kann eine dreidimensionale Rekonstruktion des Objektes berechnet werden.

Erfindungsgemäß kann das mindestens eine Röntgenschnittbild des Objektes mithilfe der Analyseeinrichtung erstellt werden. In einer Ausführungsform kann die dreidimensionale Rekonstruktion des Objektes mittels der Analyseeinrichtung erzeugt werden. Die Analyseeinrichtung kann eine Software aufweisen, unter deren Verwendung mindestens ein Röntgenschnittbild des Objektes erstellt und/oder die dreidimensionale Rekonstruktion des Objektes erzeugt werden kann.

Im Folgenden werden Ausführungsformen sowie Merkmale und Vorteile der Erfindung anhand der Figuren erläutert. Es zeigen:
- Fig. 1: eine Ausführungsform eines erfindungsgemäßen Schienensystems in einer seitlichen Ansicht,
- Fig. 2: eine Ausführungsform eines erfindungsgemäßen Schienensystems mit einem angeordneten Röntgenstrahlendetektor und einer angeordneten Röntgenstrahlenquelle in einer Ansicht von oben,
- Fig. 3: eine Ausführungsform eines Schienensystems gemäß der Erfindung mit einem Träger und einem angeordneten Objekt in einer ersten Position,
- Fig. 4: eine Ausführungsform eines Schienensystems gemäß der Erfindung mit einem Träger und einem angeordneten Objekt in einer zweiten Position,
- Fig. 5: eine Ausführungsform einer erfindungsgemäßen bildgebenden Einrichtung in einer perspektivischen Ansicht,
- Fig. 6: eine Ausführungsform einer erfindungsgemäßen bildgebenden Einrichtung in einer gekippten Position,
- Fig. 7: eine Ausführungsform einer Führungsschiene und einer an der Führungsschiene angeordneten ersten Gleitschiene gemäß der Erfindung,
- Fig. 8: eine Ausführungsform einer Führungsschiene und einer an der Führungsschiene angeordneten zweiten Gleitschiene gemäß der Erfindung,
- Fig. 9: eine Ausführungsform eines erfindungsgemäßen Schienensystems in einer perspektivischen Ansicht,
- Fig. 10: eine Ausführungsform einer erfindungsgemäßen bildgebenden Einrichtung in einer perspektivischen Ansicht,
- Fig. 11: eine Ausführungsform einer erfindungsgemäßen Führungsschiene mit an der Führungsschiene angeordneten Tragelementen in einer seitlichen Ansicht,
- Fig. 12: eine Ausführungsform einer erfindungsgemäßen Führungsschiene mit an der Führungsschiene angeordneten Tragelementen,
- Fig. 13: eine Ausführungsform eines erfindungsgemäßen Schienensystems in einem zerlegten Zustand, und
- Fig. 14: eine Ausführungsform einer erfindungsgemäßen Führungsschiene mit einer an der Führungsschiene positionierten ersten Gleitschiene in einer Detailansicht.

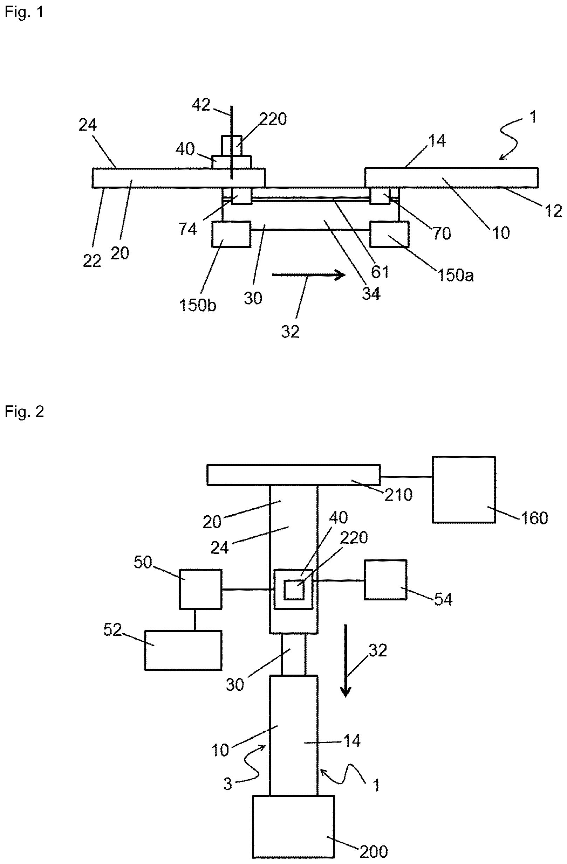

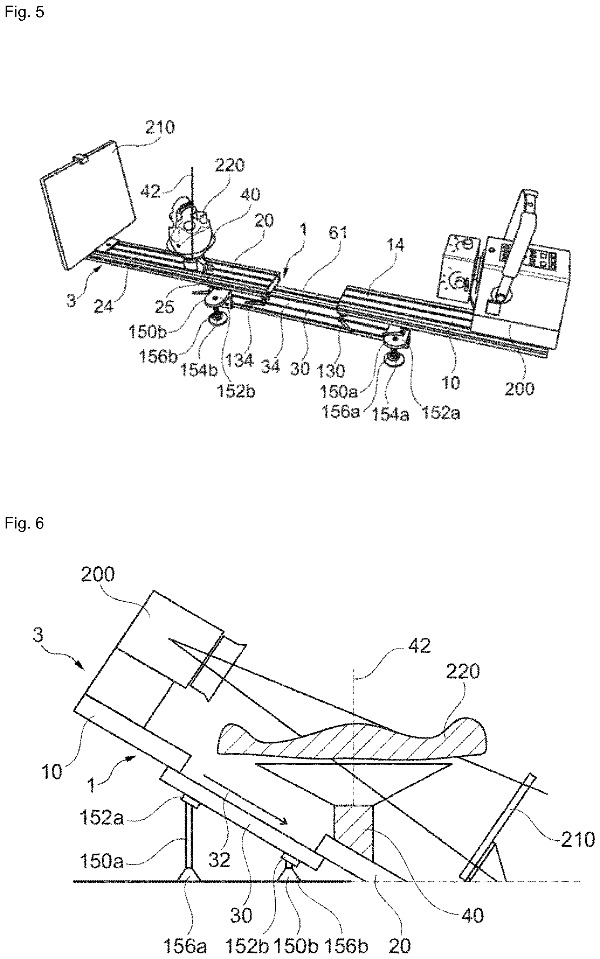

In den Figuren 1, 2, 3, 4 und 6 ist jeweils eine Ausführungsform eines erfindungsgemäßen Schienensystems 1 schematisch dargestellt. In der Figur 5 sowie den Figuren 9 bis 14 ist jeweils eine Ausführungsform des Schienensystems 1 gemäß der Erfindung gezeigt. Fig. 1 und Fig. 6 zeigen ein Schienensystem 1 gemäß der Erfindung in einer seitlichen Ansicht; die Figuren 2, 3 und 4 zeigen Ausführungsformen einer bildgebenden Einrichtung 3.

Das Schienensystem 1 kann eine Führungsschiene 30 aufweisen, die sich entlang der Längsrichtung 32 erstrecken kann. Ferner kann das Schienensystem 1 eine erste Gleitschiene 10 und eine zweite Gleitschiene 20 aufweisen. An der ersten Gleitschiene 10 kann eine Röntgenstrahlenquelle 200 angeordnet sein. An der zweiten Gleitschiene 20 kann ein Röntgenstrahlendetektor 210 angeordnet und derart positioniert sein, dass er Röntgenstrahlen, die von der Röntgenstrahlenquelle 200 ausgesandt werden können, detektieren kann. In einer Ausführungsform kann das Schienensystem 1 eine erste und/oder eine zweite Positionierungsvorrichtung 201, 211 aufweisen. Die erste Positionierungsvorrichtung 201 kann an der ersten Gleitschiene 10 positioniert sein und dazu ausgebildet sein, die Röntgenstrahlenquelle 200 zu tragen. Die zweite Positionierungsvorrichtung 211 kann an der zweiten Gleitschiene 211 angeordnet und dazu ausgebildet sein, an den Röntgenstrahlendetektor 210 anordenbar zu sein. In einer Ausführungsform kann der Röntgenstrahlendetektor 210 an der zweiten Positionierungsvorrichtung 211 festgelegt werden.

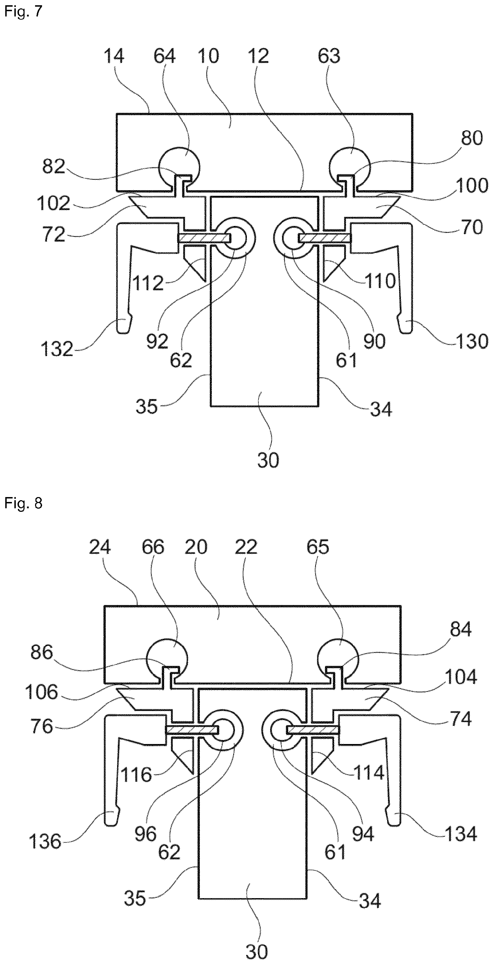

Die erste Gleitschiene 10 kann mithilfe eines ersten Tragelementes 70 und eines zweiten Tragelementes 72 an der Führungsschiene 30 angeordnet sein. Das Schienensystem 1 kann ein drittes Tragelement 74 und ein viertes Tragelement 76 aufweisen, mit dessen Hilfe die zweite Gleitschiene 20 an der Führungsschiene 30 angeordnet werden kann (Fig. 1, Fig. 9, Fig. 10, Fig. 14). In einer erfindungsgemäßen Ausführungsform kann die Führungsschiene 30 eine erste Nut 61 aufweisen (Fig. 1, Fig. 5, Figuren 9-14) und das erste Tragelement 70 und das dritte Tragelement 74 derart ausgebildet sein, dass diese an der ersten Nut 61 anordenbar sind (vergleiche auch Fig. 7 und Fig. 8).

Ein Träger 40 kann auf der zweiten Gleitschiene 20 positioniert sein. In einer Ausführungsform kann die zweite Gleitschiene 20 eine zweite Deckfläche 24 aufweisen, wobei die zweite Deckfläche 24 eine zweite Trägernut 25 aufweisen kann. Der Träger 40 kann dazu ausgebildet sein, in der zweiten Trägernut 25 positionierbar zu sein, beispielsweise mithilfe eines Trägernutensteins (Fig. 5, Fig. 9, Fig. 10, Fig. 13). In einer alternativen Ausführungsform kann die erste Gleitschiene 10 eine erste Deckfläche 14 aufweisen. Die erste Deckfläche 14 kann eine erste Trägernut aufweisen und dazu ausgebildet sein, dass der Träger 40 in der ersten Trägernut positionierbar sein kann.

Auf dem Träger 40 kann ein zu untersuchendes Objekt 220 angeordnet sein, insbesondere derart, dass das Objekt 220 zwischen der Röntgenstrahlenquelle 200 und dem Röntgenstrahlendetektor 210 positioniert sein kann (Figuren 2, 3, 4, 5, 6, 10).

Der Träger 40 kann derart ausgebildet und angeordnet sein, dass er um eine Drehachse 42 drehbar ist (Fig. 1, Fig. 5, Fig. 6). In einer Ausführungsform kann sich die Drehachse 42 senkrecht zur Längsrichtung 32 der Führungsschiene 30 erstrecken (Fig. 1, Fig. 5).

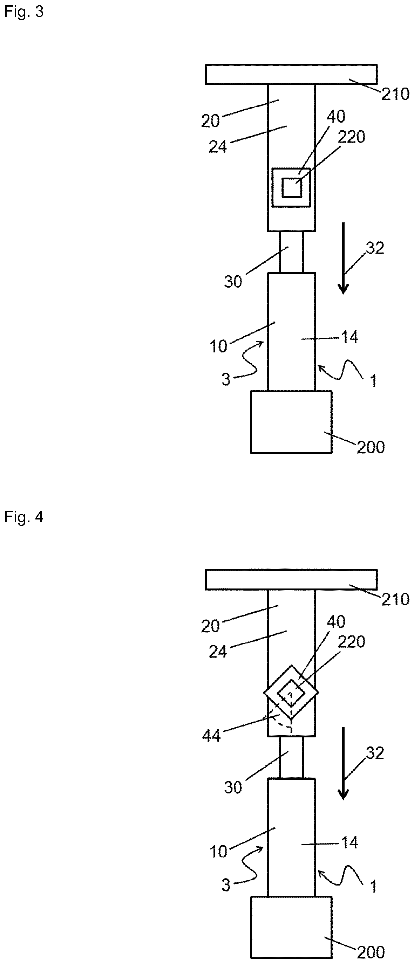

Der Träger 40 kann beispielsweise derart gedreht werden, dass das angelegte Objekt 220 aus einer ersten Position, beispielsweise der Ausgangsposition, um einen definierten Drehwinkel 44 in eine zweite Position gedreht wird (Fig. 3 und Fig. 4). Mithilfe der Röntgenstrahlenquelle 200 und des Röntgenstrahlendetektors 210 kann ein Projektionsröntgenbild des Objektes 220 in der ersten Position und der zweiten Position erzeugt werden. In einer Ausführungsform kann unter Verwendung der erzeugten Projektionsröntgenbilder des Objektes in den jeweiligen Positionen mindestens ein Röntgenschnittbild des Objektes erzeugt werden.

Erfindungsgemäß kann der Träger 40 schrittweise nacheinander gedreht werden, insbesondere derart, dass das angelegte Objekt 220 schrittweise um den definierten Drehwinkel 44 gedreht wird. Der Träger 40 kann derart gedreht werden, dass das Objekt 220 nach einer vordefinierten Anzahl an Drehschritten wieder in einer Anfangsposition positioniert ist. Dabei ist unter der Anfangsposition die Position des Objektes zu verstehen, die das Objekt einnimmt bevor ein erster Drehschritt durchgeführt wird. In einer Ausführungsform kann der definierte Drehwinkel 44 bestimmt werden aus dem Vollwinkel und der vordefinierten Anzahl an Drehschritten. In einer Ausführungsform kann die Drehung vor einem letzten Drehschritt beendet werden, wobei der letzte Drehschritt das Objekt 220 wieder in die Anfangsposition drehen kann. Für die Anfangsposition kann vor Beginn der Drehung ein Projektionsröntgenbild des Objektes 220 erzeugt werden, so dass dies am Ende der Drehung keine zusätzlichen Informationen bzw. Daten liefern würde.

Durch das Drehen des Objektes 220 können Projektionsröntgenbilder des Objektes 220 aus unterschiedlichen Richtungen erzeugt werden, wobei die Röntgenstrahlenquelle 200 und der Röntgenstrahlendetektor 210 ihre jeweiligen Positionen beibehalten können.

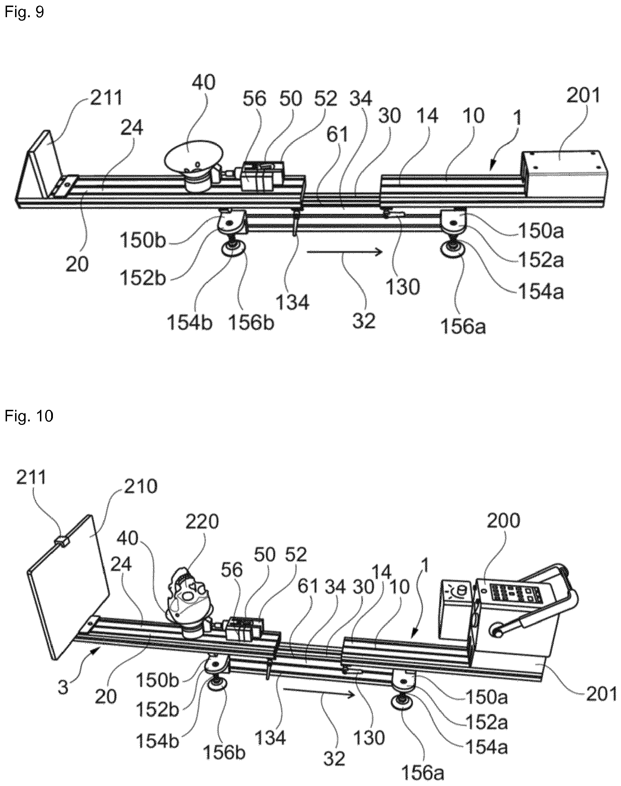

Die Drehung des Trägers 40 kann mithilfe eines Schrittmotors 50 erfolgen (Fig. 1, Fig. 9, Fig. 10, Fig. 13). In einer Ausführungsform kann der Schrittmotor 50 mithilfe einer Steuervorrichtung 52 gesteuert werden. Die Steuervorrichtung 52 und/oder der Schrittmotor 50 kann mit einer Spannungsquelle 56 verbunden sein (Fig. 9, Fig. 10).

Mittels des Schrittmotors 50 kann der Träger 40 automatisch gedreht werden, so dass das Objekt 220 um einen vordefinierten Drehwinkel 44 gedreht wird. Die Größe des vordefinierten Drehwinkels 44 und eine Wartezeit zwischen zwei aufeinanderfolgenden Drehschritten können einfach und genau eingestellt werden. In einer erfindungsgemäßen Ausführungsform ist die Steuervorrichtung 52 dazu ausgebildet, die Größe des vordefinierten Drehwinkels 44 und die Wartezeit einzustellen.

Das Schienensystem 1 kann zudem eine Analysevorrichtung 160 aufweisen (Fig. 1). Die Analysevorrichtung 160 kann beispielsweise mit dem Röntgenstrahlendetektor 210 verbunden sein und zur Bearbeitung, Auswertung und/oder Speicherung von Daten, insbesondere mindestens eines Datensatzes, ausgebildet sein. Ein Datensatz kann dabei eine Vielzahl von Datenpunkten eines Projektionsröntgenbildes aufweisen.

In einer Ausführungsform kann der Röntgenstrahlendetektor 210 eine digitale Röntgendetektorplatte aufweisen. Der Röntgenstrahlendetektor kann ein portabler Röntgenstrahlendetektor sein.

Das Schienensystem 1 kann ein erstes, ein zweites, ein drittes und ein viertes Stützelement 150a, 150b, 150c, 150d aufweisen. Die Stützelemente 150a, 150b, 150c, 150d können an der Führungsschiene 30 fixiert sein (Figuren 1, 5, 6, 9, 10, 11, 12, 13, 14). Das Schienensystem 1 kann mit den Stützelementen 150a, 150b, 150c, 150d auf einem Boden positionierbar sein (Fig. 1, Fig. 5, Fig. 9, Fig. 10). In einer Ausführungsform können die Stützelemente 150a, 150b, 150c, 150d einzeln höhenverstellbar ausgebildet sein. Dadurch können Unebenheiten des Bodens ausgeglichen werden. Insbesondere für eine Verwendung des Schienensystems 1 außerhalb von Gebäuden ist dies von Vorteil, da so auf einfache Weise das Schienensystem 1 ausgerichtet werden kann, so dass sich die angeordnete Röntgenstrahlenquelle 200, der angeordnete Röntgenstrahlendetektor 210 und das abzubildende Objekt 220 in einer definierten Anordnung zueinander befinden können.

Alternativ kann das Schienensystem 1 mittels der Stützelemente 150a, 150b, 150c, 150d gekippt werden (Fig. 6). Das bedeutet, dass bei einem gekippten Schienensystem 1, das auf einem sich in einer Bodenebene erstreckendem Boden, die Längsrichtung 32 der Führungsschiene 30 gewinkelt zur Bodenebene positioniert ist. In einer Ausführungsform kann das Schienensystem 1 gekippt werden, indem ein Abstand zwischen dem ersten Fuß 156a und dem ersten Winkelstück 152a größer ist als ein Abstand zwischen dem zweiten Fuß 156b und dem zweiten Winkelstück 152b (Fig. 6).

In einer Ausführungsform kann der Träger 40 ein Gelenk aufweisen. Mithilfe des Gelenkes kann der Träger 40 um eine Kippachse 142 gekippt werden. Die Kippachse 142 kann senkrecht zur Längsrichtung 32 der Führungsschiene 30 verlaufen.

Beispielsweise kann bei einem gekippten Schienensystem 1 mithilfe des Gelenkes der Träger 40 so positioniert werden, dass sie Drehachse 42 senkrecht zur Bodenebene verläuft (Fig. 6).

Auf diese Weise kann z. B. ein vordefinierter Bereich eines Objektes 220, beispielsweise eines großen Objektes 220, unter Verwendung des erfindungsgemäßen Schienensystems 1 abgebildet werden. Damit kann das erfindungsgemäße Schienensystem 1 auch für eine Untersuchung an großen Objekten 220 genutzt werden und ist nicht auf die Untersuchung kleiner Objekte 220 beschränkt.

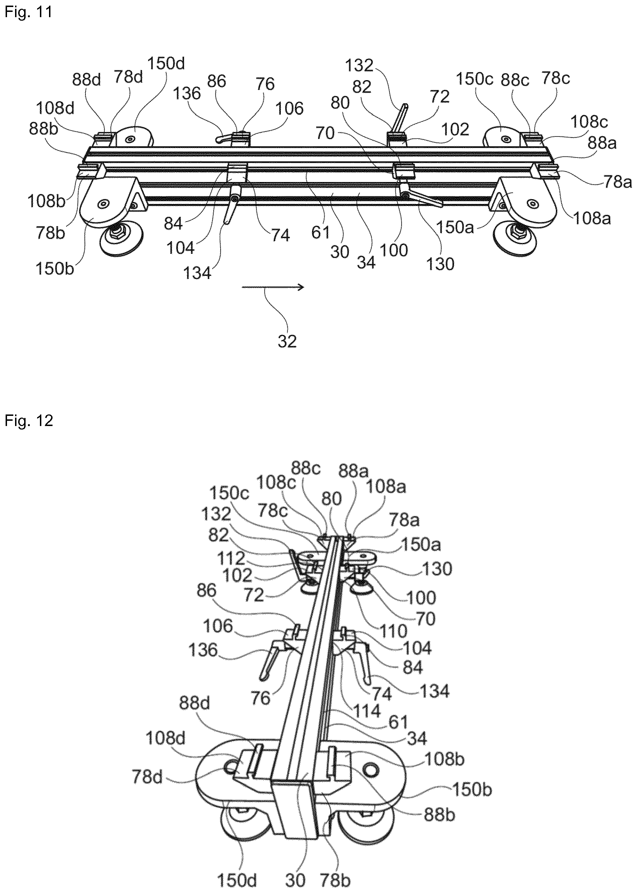

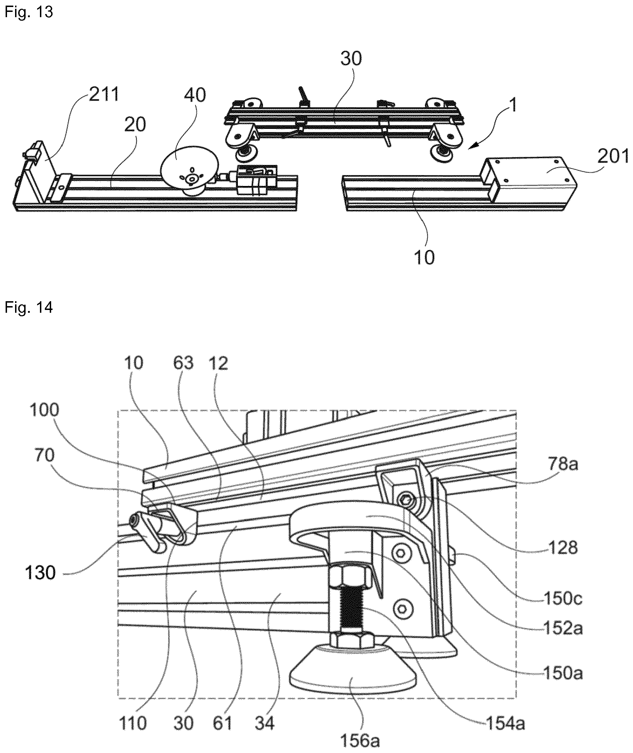

Die Figuren 7 und 8 zeigen erfindungsgemäße Ausführungsformen einer Führungsschiene 30 und einer an der Führungsschiene 30 angeordneten ersten Gleitschiene 10 (Fig. 7) sowie eine erfindungsgemäße Führungsschiene 30 und eine an der Führungsschiene 30 angeordnete zweite Gleitschiene 20 (Fig. 8). Die dargestellten schematischen Querschnitte verlaufen senkrecht zur Längsrichtung. Die Figuren 11 und 12 zeigen jeweils Ausführungsformen der Führungsschiene 30 mit an der Führungsschiene 30 positionierten Tragelementen 70, 72, 74, 76. Fig. 14 zeigt eine Detailansicht einer erfindungsgemäßen Ausführungsform einer Führungsschiene 30 an der eine erste Gleitschiene 10 festgelegt ist.

Die Führungsschiene 30 kann eine erste Seitenfläche 34 und eine zweite Seitenfläche 35 aufweisen. Die erste und die zweite Seitenfläche 34, 35 können parallel zueinander angeordnet sein. Die erste Seitenfläche 34 kann eine erste Nut 61 aufweisen, die zweite Seitenfläche 35 kann eine zweite Nut 62 aufweisen. Die erste und die zweite Nut 61, 62 können sich entlang der Längsrichtung 32 erstrecken. Die erste Gleitschiene 10 kann eine erste Bodenfläche 12 aufweisen (Fig. 7, Fig. 14). Die erste Bodenfläche 12 kann eine dritte Nut 63 und eine vierte Nut 64 aufweisen (Fig. 7). Die zweite Gleitschiene 20 kann eine zweite Bodenfläche 22 aufweisen, die eine fünfte Nut 65 und eine sechste Nut 66 aufweisen kann (Fig. 8). Die Führungsschiene 30 und die erste Gleitschiene 10 können derart zueinander positioniert sein, dass die erste und die zweite Seitenfläche 34, 35 senkrecht zur ersten Bodenfläche 12 angeordnet sind (Figuren 7, 8, 14).

In einer Ausführungsform kann das Schienensystem 1 ein erstes Tragelement 70 und ein zweites Tragelement 72 aufweisen (Fig. 7, Fig. 10, Fig. 11, Fig. 14). Das erste Tragelement 70 kann eine erste Tragfläche 100 und eine erste Anlagefläche 110 aufweisen, wobei sich die erste Tragfläche 100 senkrecht zur ersten Anlagefläche 110 erstrecken kann. Das erste Tragelement 70 kann derart in Bezug zur Führungsschiene 30 und zur ersten Gleitschiene 10 positioniert sein, dass die erste Anlagefläche 110 an der ersten Seitenfläche 34 anliegen kann und die erste Tragfläche 100 an der ersten Bodenfläche 12 anliegen kann (Fig. 7, Fig. 14). Das zweite Tragelement 72 kann eine zweite Tragfläche 102 und eine zweite Anlagefläche 112 aufweisen, die senkrecht zueinander angeordnet sein können. Das zweite Tragelement 72 kann derart in Bezug zur Führungsschiene 30 und zur ersten Gleitschiene 10 positioniert sein, dass die zweite Anlagefläche 112 an der zweiten Seitenfläche 35 anliegen kann und die zweite Tragfläche 102 an der ersten Bodenfläche 12 anliegen kann. Das bedeutet, dass mithilfe des ersten und des zweiten Tragelementes 70, 72 die erste Gleitschiene 10 an der Führungsschiene 30 angeordnet sein kann (Fig. 7).

Das erste Tragelement 70 kann einen ersten Nutenstein 80 aufweisen (Figuren 7, 11, 12). Der erste Nutenstein 80 kann in der dritten Nut 63 angeordnet sein. Mithilfe eines ersten Befestigungsmittels 120 kann der erste Nutenstein 80 in der dritten Nut 63 fixiert werden. Das erste Befestigungsmittel 120 kann lösbar sein, so dass der erste Nutenstein 80 gelöst und beispielsweise in der dritten Nut 63 bewegbar ist (Fig. 7).

Erfindungsgemäß kann in einer Ausführungsform das zweite Tragelement 72 einen zweiten Nutenstein 82 aufweisen. Der zweite Nutenstein 82 kann in der vierten Nut 64 positioniert sein und dort beispielsweise mit einem zweiten Befestigungsmittel 122 lösbar fixiert sein.

Ferner kann ein erstes Klemmelement 90 am ersten Tragelement 70 sowie in der ersten Nut 61 angeordnet sein (Fig. 7, Fig. 14). Das erste Klemmelement 90 kann sich in der ersten Nut 61 verklemmen, so dass das erste Tragelement 70 an der ersten Nut 61 fixiert sein kann. Das erste Klemmelement 90 kann einen ersten Hebel 130 aufweisen, wobei mithilfe des ersten Hebels 130 das erste Klemmelement 90 von einem ersten Zustand in einen zweiten Zustand (bzw. von einem zweiten in einen ersten Zustand) überführt werden kann, wobei das erste Klemmelement 90 im ersten Zustand in der ersten Nut 61 verklemmt ist und das erste Klemmelement 90 im zweiten Zustand in der ersten Nut 61 bewegbar (nicht verklemmt) ist.

Entsprechend kann das Schienensystem 1 ein zweites Klemmelement 92 aufweisen, das am zweiten Tragelement 72 angeordnet sein kann. Das zweite Klemmelement 92 kann sich in der zweiten Nut 62 verklemmen, so dass das zweite Tragelement 72 an der zweiten Nut 62 fixiert sein kann. Das zweite Klemmelement 92 kann einen zweiten Hebel 132 aufweisen. Mithilfe des zweiten Hebels 132 kann das zweite Klemmelement 92 von einem ersten Zustand, in dem das zweite Klemmelement 92 in der zweiten Nut 62 verklemmt ist, in einen zweiten Zustand überführt werden, in dem das zweite Klemmelement 92 in der zweiten Nut 62 bewegbar ist (bzw. kann das zweite Klemmelement 92 vom zweiten in den ersten Zustand überführt werden) (Fig. 7).

Erfindungsgemäß kann das Schienensystem 1 ein drittes und ein viertes Tragelement 74, 76 aufweisen (Fig. 8, Fig. 11, Fig. 12). Das dritte und das vierte Tragelement 74, 76 können analog zum ersten und zum zweiten Tragelement 70, 72 ausgebildet sein und an der zweiten Gleitschiene 20 angeordnet sein, so dass mithilfe des dritten und das vierten Tragelementes 74, 76 die zweite Gleitschiene 20 an der Führungsschiene 30 angeordnet sein kann.

Das dritte Tragelement 74 kann mithilfe eines dritten Nutensteins 84 in der fünften Nut 65 positioniert sein und mithilfe eines dritten Klemmelementes 94 in der ersten Nut 61 verklemmbar sein. Entsprechend kann das vierte Tragelement 76 mithilfe eines vierten Nutensteins 86 in der sechsten Nut 66 positioniert sein und mithilfe eines vierten Klemmelementes 96 in der zweiten Nut 62 verklemmbar sein. Die zweite Bodenfläche 22 kann an der dritten Tragfläche 104 des dritten Tragelementes 74 und an der vierten Tragfläche 106 des vierten Tragelementes 76 anliegen. Die Führungsschiene 30 kann an der dritten und der vierten Anlagefläche 114, 116 anliegen.

Das dritte Klemmelement 94 kann mithilfe eines dritten Hebels 134 und das vierte Klemmelement 96 mithilfe eines vierten Hebels 136 jeweils vom ersten in den zweiten Zustand überführt werden.

Mithilfe des ersten und zweiten Tragelementes 70, 72, der ersten und zweiten Klemmeinheit 90, 92 und des ersten und zweiten Nutensteins 80, 82 die erste Gleitschiene 10 an der Führungsschiene 30 fixiert werden kann. Unter Verwendung des dritten und des vierten Tragelementes 74, 76 kann die zweite Gleitschiene 20 an der Führungsschiene 30 festgelegt werden.

Die Fixierungen können zudem auf einfache Weise gelöst werden, beispielsweise indem die jeweiligen Klemmeinheiten 90, 92, 94, 96 in den zweiten Zustand (in der jeweiligen Nut 61, 62 bewegbar) überführt werden, so dass die erste und die zweite Gleitschiene 10, 20 bewegbar sein und entlang der Führungsschiene 30 verschoben werden können. Weiterhin können die erste und die zweite Gleitschiene 10, 20 auf einfach Weise von der Führungsschiene 30 entfernt werden, so dass die erste Gleitschiene 10, die zweite Gleitschiene 20 und die Führungsschiene 30 als einzelne Bauteile vorliegen können (siehe Fig. 13), wodurch beispielsweise ein Transport des Schienensystems 1 erleichtert werden kann.

Weiterhin kann das Schienensystem 1 einfach zusammengebaut werden durch ein einfaches Zusammenschieben der ersten Gleitschiene 10, der zweiten Gleitschiene 20 und der Führungsschiene 30.

In einer Ausführungsform kann das Schienensystem 1 weitere Tragelemente 78a, 78b, 78c, 78d aufweisen (Figuren 11, 12, 13, 14). Diese können mithilfe von weiteren Befestigungsmitteln 128 an der Führungsschiene 30 fixiert sein (Fig. 14). Die weiteren Tragelemente 78a, 78b, 78c, 78d können weitere Nutensteine 88a, 88b, 88c, 88d sowie weitere Tragflächen 108a, 108b, 108c, 108d aufweisen (Fig. 11, Fig. 12). Die weiteren Tragelemente 78a, 78b, 78c, 78d können zur Anlage an der ersten Gleitschiene 10 (Fig. 14) oder der zweiten Gleitschiene 20 ausgebildet sein und können zur Stabilisierung der Position der jeweiligen Gleitschiene 10, 20 dienen.

## Patentansprüche

1. Schienensystem (1) zum Anordnen einer Röntgenstrahlenquelle (200) bezüglich eines Röntgenstrahlendetektors (210) und eines abzubildenden Objekts (220) mit:
- einer ersten Gleitschiene (10), die dazu ausgebildet ist, eine Röntgenstrahlquelle (200) zu tragen,
- einer zweiten Gleitschiene (20), die dazu ausgebildet ist, einen Röntgenstrahlendetektor (210) zu tragen,
- einer Führungsschiene (30), die zum Führen der ersten und der zweiten Gleitschiene (10, 20) ausgebildet ist, wobei die die erste und die zweite Gleitschiene (10, 20) jeweils dazu ausgebildet sind, zum Positionieren der Röntgenstrahlenquelle (200) bezüglich des Röntgenstrahlendetektors (210) zueinander verschiebbar auf der Führungsschiene (30) angeordnet zu werden, wobei die erste und die zweite Gleitschiene (10, 20) lösbar an der Führungsschiene (30) festlegbar sind, um eine Position der Röntgenstrahlenquelle (200) bezüglich des Röntgenstrahlendetektors (210) zu fixieren, und
- einem Träger (40), der zum Tragen des mittels Röntgenstrahlung der Röntgenstrahlenquelle (200) abzubildenden Objekts (220) ausgebildet ist, wobei der Träger (40) weiterhin zum Positionieren des Trägers (40) zwischen der Röntgenstrahlenquelle (200) und dem Röntgenstrahlendetektor (210) dazu ausgebildet ist, verschiebbar auf der ersten Gleitschiene (10) oder der zweiten Gleitschiene (20) angeordnet zu werden, wobei der Träger (40) an der jeweiligen Gleitschiene (10, 20) festlegbar ist, um eine Position des Trägers (40) bezüglich der Röntgenstrahlenquelle (200) und des Röntgenstrahlendetektors (210) zu fixieren.

2. Schienensystem (1) nach Anspruch 1, **dadurch gekennzeichnet, dass** sich die Führungsschiene (30) entlang einer Längsrichtung (32) erstreckt und die erste Gleitschiene (10) und/oder die zweite Gleitschiene (20) entlang der Längsrichtung (32) bezüglich der Führungsschiene (30) verschiebbar sind, so dass eine Position der Röntgenstrahlenquelle (200) bezüglich des Röntgenstrahlendetektors (210) einstellbar ist.

3. Schienensystem (1) nach Anspruch 2, **dadurch gekennzeichnet, dass** der Träger (40) bezüglich der ersten oder der zweiten Gleitschiene (10, 20) um eine Drehachse (42) drehbar ist, wobei sich die Drehachse (42) orthogonal zur Längsrichtung (32) der Führungsschiene (30) erstreckt, so dass ein auf dem Träger (40) angeordnetes Objekt (220) um einen definierten Drehwinkel (44) aus einer ersten Position in eine zweite Position drehbar ist, oder so, dass ein auf dem Träger (40) angeordnetes Objekt (220) aus einer Anfangsposition sukzessive in einer Vielzahl an Drehschritten um jeweils einen definierten Drehwinkel (44) drehbar ist, so dass nach einer definierten Anzahl an Drehschritten das Objekt (220) wieder in der Anfangsposition ist, insbesondere wobei das Schienensystem (1) einen Schrittmotor (50) aufweist, der dazu ausgebildet ist, den Träger (40) um die Drehachse (42) zu drehen, insbesondere wobei das Schienensystem (1) eine Steuervorrichtung (52) aufweist, die dazu ausgebildet ist, den Schrittmotor (50) anzusteuern.

4. Schienensystem (1) nach einem der Ansprüche 2 oder 3, **dadurch gekennzeichnet, dass** das Schienensystem (1) einen Aktuator (54) aufweist, der dazu ausgebildet ist, den Träger (40) entlang der Längsrichtung (32) zu bewegen.

5. Schienensystem (1) nach einem der Ansprüche 2 bis 4, **dadurch gekennzeichnet, dass**
die Führungsschiene (30) eine erste Seitenfläche (34) und eine von der ersten Seitenfläche (34) abgewandte zweite Seitenfläche (35) aufweist, wobei die erste Seitenfläche (34) eine erste Nut (61) und die zweite Seitenfläche (35) eine zweite Nut (62) aufweist, wobei sich die erste Nut (61) und die zweite Nut (62) entlang der Längsrichtung (32) erstrecken, wobei
die erste Gleitschiene (10) eine erste Bodenfläche (12) aufweist, wobei die erste Bodenfläche (12) eine dritte Nut (63) und eine vierte Nut (64) aufweist, wobei sich die dritte Nut (63) und die vierte Nut (64) entlang der Längsrichtung (32) erstrecken, wobei
die zweite Gleitschiene (20) eine zweite Bodenfläche (22) aufweist, wobei die zweite Bodenfläche (22) eine fünfte Nut (65) und eine sechste Nut (66) aufweist, wobei sich die fünfte Nut (65) und die sechste Nut (66) entlang der Längsrichtung (32) erstrecken, wobei
das Schienensystem (1) zumindest ein erstes Tragelement (70), ein zweites Tragelement (72), ein drittes Tragelement (74) und ein viertes Tragelement (76) aufweist, wobei
das Schienensystem (1) zumindest einen ersten Nutenstein (80), einen zweiten Nutenstein (82), einen dritten Nutenstein (84) und einen vierten Nutenstein (86) aufweist, wobei der erste Nutenstein (80) in der dritten Nut (63), der zweite Nutenstein (82) in der vierten Nut (64), der dritte Nutenstein (84) in der fünften Nut (65) und der vierte Nutenstein (86) in der sechsten Nut (66) anordenbar sind, und wobei der erste Nutenstein (80) am ersten Tragelement (70), der zweite Nutenstein (82) am zweiten Tragelement (72), der dritte Nutenstein (84) am dritten Tragelement (74) und der vierte Nutenstein (86) am vierten Tragelement (76) festlegbar sind, und wobei
ein erstes Klemmelement (90) am ersten Tragelement (70), ein zweites Klemmelement (92) am zweiten Tragelement (72), ein drittes Klemmelement (94) am dritten Tragelement (74) und ein viertes Klemmelement (96) am vierten Tragelement (76) angeordnet sind, wobei
das erste Klemmelement (90) und das dritte Klemmelement (94) in der ersten Nut anordenbar (61) sind, wobei
das zweite Klemmelement (92) und das vierte Klemmelement (96) in der zweiten Nut anordenbar (62) sind,
so dass die erste Gleitschiene (10) und die zweite Gleitschiene (20) mit der Führungsschiene (30) verbindbar sind.

6. Schienensystem (1) nach Anspruch 5, **dadurch gekennzeichnet, dass** das erste Klemmelement (90) in der ersten Nut (61) verklemmbar ist, so dass das erste Tragelement (70) an der Führungsschiene (30) fixierbar ist, und/oder dass das zweite Klemmelement (92) in der zweiten Nut (62) verklemmbar ist, so dass das zweite Tragelement (72) an der Führungsschiene (30) fixierbar ist, und/oder dass das dritte Klemmelement (94) in der ersten Nut (61) verklemmbar ist, so dass das dritte Tragelement (74) an der Führungsschiene (30) fixierbar ist, und/oder dass das vierte Klemmelement (96) in der zweiten Nut (62) verklemmbar ist, so dass das vierte Tragelement (76) an der Führungsschiene (30) fixierbar ist, und/oder, dass das erste Tragelement (70) eine erste Tragfläche (100) und eine erste Anlagefläche (110) aufweist, wobei die erste Tragfläche (100) und die erste Anlagefläche (110) orthogonal zueinander angeordnet sind, und/oder dass das zweite Tragelement (72) eine zweite Tragfläche (102) und eine zweite Anlagefläche (112) aufweist, wobei die zweite Tragfläche (102) und die zweite Anlagefläche (112) orthogonal zueinander angeordnet sind, und/oder dass das dritte Tragelement (74) eine dritte Tragfläche (104) und eine dritte Anlagefläche (114) aufweist, wobei die dritte Tragfläche (104) und die dritte Anlagefläche (114) orthogonal zueinander angeordnet sind, und/oder dass das vierte Tragelement (76) eine vierte Tragfläche (106) und eine vierte Anlagefläche (116) aufweist, wobei die vierte Tragfläche (106) und die vierte Anlagefläche (116) orthogonal zueinander angeordnet sind, insbesondere wobei das erste Tragelement (70) derart in Bezug zur Führungsschiene (30) und zur ersten Gleitschiene (10) positionierbar ist, dass die erste Tragfläche (100) an der ersten Bodenfläche (12) anliegt und die erste Anlagefläche (110) an der ersten Seitenfläche (34) anliegt, und/oder dass das zweite Tragelement (72) derart in Bezug zur Führungsschiene (30) und zur ersten Gleitschiene (10) positionierbar ist, dass die zweite Tragfläche (102) an der ersten Bodenfläche (12) anliegt und die zweite Anlagefläche (112) an der zweiten Seitenfläche (35) anliegt, und/oder dass das dritte Tragelement (74) derart in Bezug zur Führungsschiene (30) und zur zweiten Gleitschiene (20) positionierbar ist, dass die dritte Tragfläche (104) an der zweiten Bodenfläche (22) anliegt und die dritte Anlagefläche (114) an der ersten Seitenfläche (34) anliegt, und/oder dass das vierte Tragelement (76) derart in Bezug zur Führungsschiene (30) und zur zweiten Gleitschiene (20) positionierbar ist, dass die vierte Tragfläche (106) an der zweiten Bodenfläche (22) anliegt und die vierte Anlagefläche (116) an der zweiten Seitenfläche (35) anliegt.

7. Schienensystem (1) nach einem der Ansprüche 5 bis 6, **dadurch gekennzeichnet, dass** das Schienensystem (1) ein erstes Befestigungsmittel aufweist, das dazu ausgebildet ist, den ersten Nutenstein (80) in der dritten Nut (63) festzulegen, und/oder ein zweites Befestigungsmittel aufweist, das dazu ausgebildet ist, den zweiten Nutenstein (82) in der vierten Nut (64) festzulegen, und/oder ein drittes Befestigungsmittel aufweist, das dazu ausgebildet ist, den dritten Nutenstein (84) in der fünften Nut (65) festzulegen, und/oder ein viertes Befestigungsmittel aufweist, das dazu ausgebildet ist, den vierten Nutenstein (86) in der sechsten Nut (66) festzulegen, und/oder, dass die Führungsschiene (30), die erste Gleitschiene (10) und die zweite Gleitschiene (20) dazu ausgebildet sind, derart zueinander angeordnet zu werden, dass die erste und die zweite Seitenfläche (34, 35) senkrecht zur ersten Bodenfläche (12) verlaufen und die erste und die zweite Seitenfläche (34, 35) senkrecht zur zweiten Bodenfläche (22) verlaufen.

8. Schienensystem (1) nach einem der Ansprüche 2 bis 7, **dadurch gekennzeichnet, dass** der Träger (40) einen Trägernutenstein aufweist und die erste Gleitschiene (10) eine erste Deckfläche (14) aufweist, wobei die erste Deckfläche (14) eine erste Trägernut (15) aufweist, wobei sich die erste Trägernut (15) entlang der Längsrichtung (32) erstreckt, und wobei der Trägernutenstein dazu ausgebildet ist, in der ersten Trägernut (15) angeordnet zu werden, und/oder dass die zweite Gleitschiene (20) eine zweite Deckfläche (24) aufweist, wobei die zweite Deckfläche (24) eine zweite Trägernut (25) aufweist, wobei sich die zweite Trägernut (25) entlang der Längsrichtung (32) erstreckt, und wobei der Trägernutenstein dazu ausgebildet ist, in der zweiten Trägernut (25) angeordnet zu werden, und/oder dass der Träger (40) ein Gelenk aufweist, so dass der Träger (40) um eine Kippachse (142) kippbar ist, wobei die Kippachse (142) orthogonal zur Längsrichtung (32) der Führungsschiene (30) verläuft.

9. Schienensystem (1) nach einem der vorherigen Ansprüche, **dadurch gekennzeichnet, dass** das Schienensystem (1) eine Mehrzahl an Stützelementen (150) aufweist, wobei das jeweilige Stützelement (150) ein Winkelstück (152), eine am Winkelstück (152) anordbare Verbindungsstange (154) und einen an der Verbindungsstange (154) angeordneten Fuß (156) aufweist, wobei das Winkelstück (152) dazu ausgebildet ist, an der Führungsschiene (30) festgelegt zu werden, und wobei mithilfe der Verbindungsstange (154) ein Abstand zwischen dem Winkelstück (152) und dem Fuß (156) einstellbar ist.

10. Schienensystem (1) nach einem der vorherigen Ansprüche, **dadurch gekennzeichnet, dass** die erste Gleitschiene (10), die zweite Gleitschiene (20) und der Träger (40) lösbar an der Führungsschiene (30) festlegbar sind, so dass das Schienensystem (1) in die erste Gleitschiene (10), die zweite Gleitschiene (20), den Träger (40) und die Führungsschiene (30) zerlegbar ist.

11. Schienensystem (1) nach einem der vorherigen Ansprüche, **dadurch gekennzeichnet, dass** das Schienensystem (1) eine Analyseeinrichtung (160) aufweist, wobei die Analyseeinrichtung (160) zur Bearbeitung, Auswertung und/oder Speicherung eines Datensatzes ausgebildet ist, insbesondere wobei die Analyseeinrichtung (160) dazu ausgebildet ist, einen Transformationsalgorithmus auszuführen, der anhand einer Vielzahl von Projektionsröntgenbildern mindestens ein Röntgenschnittbild des Objektes (220) erstellt.

12. Bildgebende Einrichtung (3) zum Erzeugen von Projektionsröntgenbildern eines Objekts (220), wobei die bildgebende Einrichtung (3) ein Schienensystem (1) nach einem der vorhergehenden Ansprüche aufweist, sowie ferner eine Röntgenstrahlenquelle (200) und einen Röntgenstrahlendetektor (210), wobei die erste Gleitschiene (10) dazu ausgebildet ist, die Röntgenstrahlenquelle (200) zu tragen, und wobei die zweite Gleitschiene (20) dazu ausgebildet ist, den Röntgenstrahlendetektor (210) zu tragen.

13. Bildgebende Einrichtung (3) nach Anspruch 12, **dadurch gekennzeichnet, dass** der Röntgenstrahlendetektor (210) eine digitale Röntgenstrahlendetektorplatte aufweist, und/oder dass die Röntgenstrahlenquelle (200) eine portable Röntgenstrahlenquelle ist, und/oder dass die bildgebende Einrichtung eine Analyseeinrichtung (160) aufweist, wobei die Analyseeinrichtung (160) dazu ausgebildet ist, einen Transformationsalgorithmus auszuführen, der anhand einer Vielzahl von Projektionsröntgenbildern mindestens ein Röntgenschnittbild des Objektes (220) erstellt.

14. Verfahren zum Erzeugen eines Projektionsröntgenbildes eines Objekts (220) unter Verwendung eines Schienensystems (1) gemäß einem der Ansprüche 1 bis 11 oder unter Verwendung einer bildgebenden Einrichtung (3) gemäß einem der Ansprüche 12 bis 13, wobei ein Objekt (220) auf dem Träger (40) des Schienensystems (1) angeordnet wird und mittels der an der ersten Gleitschiene (10) festgelegten Röntgenstrahlenquelle (200) und des an der zweiten Gleitschiene (20) festgelegten Röntgenstrahlendetektors (210) zumindest ein Projektionsröntgenbild des Objekts (220) erzeugt wird.

15. Verfahren nach Anspruch 14, **dadurch gekennzeichnet, dass** das auf dem Träger (40) angeordnete Objekt (220) mittels des Schienensystems (1) aus einer ersten Position in eine zweite Position gedreht wird und ein erstes Projektionsröntgenbild des Objektes (220) in der ersten Position und ein zweites Projektionsröntgenbild des Objektes (220) in der zweiten Position erzeugt wird, insbesondere wobei aus einer Vielzahl an Projektionsröntgenbildern mindestens ein Röntgenschnittbild des Objektes (220) erstellt wird.

## Claims

1. A rail system (1) for arranging an X-ray source (200) with respect to an X-ray detector (210) and an object (220) to be imaged, comprising:
- a first slide rail (10) adapted to support an X-ray source (200),
- a second slide rail (20) adapted to support an X-ray detector (210),
- a guide rail (30) adapted to guide the first and second slide rails (10, 20), wherein the first and second slide rails (10, 20) are each adapted to be slidably arranged on the guide rail (30) relative to each other for positioning the X-ray source (200) with respect to the X-ray detector (210), the first and second slide rails (10, 20) being detachably fixable to the guide rail (30) for fixing a position of the X-ray source (200) with respect to the X-ray detector (210), and
- a carrier (40) adapted to carry the object (220) to be imaged by means of X-rays from the X-ray source (200), the carrier (40) being further adapted to be slidably arranged on the first slide rail (10) or on the second slide rail (20) for positioning the carrier (40) between the X-ray source (200) and the X-ray detector (210), wherein the carrier (40) is fixable to the respective slide rail (10, 20) to fix a position of the carrier (40) with respect to the X-ray source (200) and the X-ray detector (210).

2. The rail system (1) according to claim 1, **characterized in that** the guide rail (30) extends along a longitudinal direction (32) and the first slide rail (10) and/or the second slide rail (20) are displaceable along the longitudinal direction (32) with respect to the guide rail (30) so that a position of the X-ray source (200) with respect to the X-ray detector (210) is adjustable.

3. The rail system (1) according to claim 2, **characterized in that** the carrier (40) is rotatable about an axis of rotation (42) with respect to the first or the second slide rail (10, 20), the axis of rotation (42) extending orthogonally to the longitudinal direction (32) of the guide rail (30), so that an object (220) arranged on the carrier (40) is rotatable through a defined angle of rotation (44) from a first position to a second position, or so, that an object (220) arranged on the carrier (40) is rotatable from an initial position successively in a plurality of rotational steps by a defined rotational angle (44) in each case, so that after a defined number of rotational steps the object (220) is again in the initial position, in particular wherein the rail system (1) has a stepper motor (50) which is designed to rotate the carrier (40) about the axis of rotation (42), in particular wherein the rail system (1) has a control device (52) which is designed to control the stepper motor (50).

4. The rail system (1) according to one of the claims 2 or 3, **characterized in that** the rail system (1) has an actuator (54) which is designed to move the carrier (40) along the longitudinal direction (32).

5. The rail system (1) according to one of claims 2 to 4, **characterized in that**
the guide rail (30) has a first side surface (34) and a second side surface (35) facing away from the first side surface (34), the first side surface (34) having a first groove (61) and the second side surface (35) having a second groove (62), the first groove (61) and the second groove (62) extending along the longitudinal direction (32), wherein
said first slide rail (10) having a first bottom surface (12), said first bottom surface (12) having a third groove (63) and a fourth groove (64), said third groove (63) and said fourth groove (64) extending along the longitudinal direction (32), wherein
the second slide rail (20) has a second bottom surface (22), the second bottom surface (22) having a fifth groove (65) and a sixth groove (66), the fifth groove (65) and the sixth groove (66) extending along the longitudinal direction (32), wherein
the rail system (1) comprises at least a first support element (70), a second support element (72), a third support element (74) and a fourth support element (76), wherein
the rail system (1) comprises at least a first groove block (80), a second groove block (82), a third groove block (84) and a fourth groove block (86), wherein the first groove block (80) is located in the third groove (63), the second groove block (82) is located in the fourth groove (64), the third groove block (84) being arrangeable in the fifth groove (65) and the fourth groove block (86) being arrangeable in the sixth groove (66), and wherein the first groove block (80) is fixable to the first support element (70), the second groove block (82) is fixable to the second support element (72), the third groove block (84) is fixable to the third support element (74) and the fourth groove block (86) is fixable to the fourth support element (76), and wherein
a first clamping element (90) is arranged on the first support element (70), a second clamping element (92) is arranged on the second support element (72), a third clamping element (94) is arranged on the third support element (74), and a fourth clamping element (96) is arranged on the fourth support element (76), wherein
the first clamping element (90) and the third clamping element (94) are arrangeable in the first groove (61), wherein
the second clamping element (92) and the fourth clamping element (96) are arrangeable in the second groove (62),
so that the first slide rail (10) and the second slide rail (20) can be connected to the guide rail (30).

6. The rail system (1) according to claim 5, **characterized in that** the first clamping element (90) can be clamped in the first groove (61), so that the first support element (70) can be fixed to the guide rail (30), and/or **in that** the second clamping element (92) can be clamped in the second groove (62), so that the second support element (72) can be fixed to the guide rail (30), and/or **in that** the third clamping element (94) can be clamped in the first groove (61), so that the third support element (74) can be fixed to the guide rail (30), and/or **in that** the fourth clamping element (96) can be clamped in the second groove (62), so that the fourth support element (76) can be fixed to the guide rail (30), and/or **in that** the first support element (70) has a first support surface (100) and a first abutment surface (110), the first support surface (100) and the first abutment surface (110) being arranged orthogonally to one another, and/or **in that** the second support element (72) has a second support surface (102) and a second abutment surface (112) wherein the second support surface (102) and the second abutment surface (112) are arranged orthogonally to each other, and/or that the third support element (74) comprises a third support surface (104) and a third abutment surface (114), wherein the third support surface (104) and the third abutment surface (114) are arranged orthogonally to each other, and/or **in that** the fourth support element (76) has a fourth support surface (106) and a fourth abutment surface (116), the fourth support surface (106) and the fourth abutment surface (116) being arranged orthogonally with respect to one another, in particular the first support element (70) being positionable in such a way with respect to the guide rail (30) and to the first slide rail (10), **in that** the first support surface (100) bears against the first bottom surface (12) and the first support surface (110) bears against the first side surface (34), and/or in that the second support element (72) can be positioned in such a way with respect to the guide rail (30) and to the first slide rail (10) **in that** the second support surface (102) bears against the first bottom surface (12) and the second support surface (112) bears against the second side surface (35), and/or **in that** the third support element (74) can be positioned in relation to the guide rail (30) and to the second slide rail (20) in such a way that the third support surface (104) bears against the second bottom surface (22) and the third support surface (114) bears against the first side surface (34), and/or **in that** the fourth support element (76) can be positioned in relation to the guide rail (30) and to the second slide rail (20) in such a way that the fourth support surface (106) bears against the second bottom surface (22) and the fourth support surface (116) bears against the second side surface (35).

7. The rail system (1) according to any one of claims 5 to 6, **characterized in that** the rail system (1) comprises a first fastening means adapted to fix the first groove block (80) in the third groove (63), and/or comprises a second fastening means adapted to fix the second groove block (82) in the fourth groove (64), and/or comprises a third fastening means adapted to fix the third groove block (84) in the fifth groove (65), and/or a fourth fastening means adapted to fix the fourth groove block (86) in the sixth groove (66), and/or **in that** the guide rail (30), the first slide rail (10) and the second slide rail (20) are adapted to be arranged with respect to each other in such a way that the first and the second side surfaces (34, 35) are perpendicular to the first bottom surface (12) and the first and the second side surfaces (34, 35) are perpendicular to the second bottom surface (22).

8. The rail system (1) according to any one of claims 2 to 7, **characterized in that** the carrier (40) comprises a carrier groove block and the first slide rail (10) comprises a first cover surface (14), wherein the first cover surface (14) comprises a first carrier groove (15), wherein the first carrier groove (15) extends along the longitudinal direction (32), and wherein the carrier groove block is adapted to be arranged in the first carrier groove (15), and/or **in that** the second slide rail (20) comprises a second cover surface (24), wherein the second cover surface (24) comprises a second carrier groove (25), wherein the second carrier groove (25) extends along the longitudinal direction (32), and wherein the carrier groove block is adapted to be arranged in the second carrier groove (25), and/or that the carrier (40) comprises a joint, so that the carrier (40) is tiltable about a tilting axis (142), wherein the tilting axis (142) extends orthogonally to the longitudinal direction (32) of the guide rail (30).

9. The rail system (1) according to one of the preceding claims, **characterized in that** the rail system (1) has a plurality of support elements (150), the respective support element (150) having an angle piece (152), a connecting rod (154) which can be arranged on the angle piece (152), and a foot (156) arranged on the connecting rod (154), wherein the angle piece (152) is designed to be fixed to the guide rail (30), and wherein a distance between the angle piece (152) and the foot (156) can be adjusted with the aid of the connecting rod (154).

10. The rail system (1) according to one of the preceding claims, **characterized in that** the first slide rail (10), the second slide rail (20) and the carrier (40) can be detachably fixed to the guide rail (30), so that the rail system (1) can be disassembled into the first slide rail (10), the second slide rail (20), the carrier (40) and the guide rail (30).

11. The rail system (1) according to one of the preceding claims, **characterized in that** the rail system (1) has an analysis device (160), the analysis device (160) being designed for processing, evaluating and/or storing a data set, in particular the analysis device (160) being designed to execute a transformation algorithm which uses a plurality of projection X-ray images to produce at least one X-ray sectional image of the object (220).

12. An imaging device (3) for generating projection X-ray images of an object (220), wherein the imaging device (3) comprises a rail system (1) according to one of the preceding claims, and further comprises an X-ray source (200) and an X-ray detector (210), wherein the first slide rail (10) is adapted to support the X-ray source (200), and wherein the second slide rail (20) is adapted to support the X-ray detector (210).

13. The imaging device (3) according to claim 12, **characterized in that** the X-ray detector (210) comprises a digital X-ray detector plate, and/or **in that** the X-ray source (200) is a portable X-ray source, and/or **in that** the imaging device comprises an analysis device (160), the analysis device (160) being adapted to execute a transformation algorithm which produces at least one X-ray sectional image of the object (220) on the basis of a plurality of projection X-ray images.

14. A method for generating a projection X-ray image of an object (220) using a rail system (1) according to one of claims 1 to 11 or using an imaging device (3) according to one of claims 12 to 13, wherein an object (220) is arranged on the carrier (40) of the rail system (1) and at least one projection X-ray image of the object (220) is generated by means of the X-ray source (200) fixed to the first slide rail (10) and the X-ray detector (210) fixed to the second slide rail (20).

15. The method according to claim 14, **characterized in that** the object (220) arranged on the carrier (40) is rotated by means of the rail system (1) from a first position into a second position and a first projection X-ray image of the object (220) in the first position and a second projection X-ray image of the object (220) in the second position are generated, in particular wherein at least one X-ray sectional image of the object (220) is generated from a plurality of projection X-ray images.

## Revendications

1. Système de rail (1) pour l'agencement d'une source de rayons X (200) par rapport à un détecteur de rayons X (210) et un objet à imager (220) avec :
- un premier rail coulissant (10) qui est réalisé pour porter une source de rayons X (200),
- un second rail coulissant (20) qui est réalisé pour porter un détecteur de rayons X (210),
- un rail de guidage (30) qui est réalisé en vue de guider le premier et le second rail coulissant (10, 20), dans lequel le premier et le second rail coulissant (10, 20) sont chacun réalisés pour être agencés de manière coulissante l'un par rapport à l'autre sur le rail de guidage (30) en vue de positionner la source de rayons X (200) par rapport au détecteur de rayons X (210), dans lequel le premier et le second rail coulissant (10, 20) peuvent être immobilisés de manière amovible sur le rail de guidage (30) pour fixer une position de la source de rayons X (200) par rapport au détecteur de rayons X (210), et
- un support (40) qui est réalisé en vue de porter l'objet (220) à imager au moyen du rayonnement X de la source de rayons X (200), dans lequel le support (40) est en outre réalisé en vue de positionner le support (40) entre la source de rayons X (200) et le détecteur de rayons X (210) pour être agencé de manière coulissante sur le premier rail coulissant (10) ou le second rail coulissant (20), dans lequel le support (40) peut être immobilisé sur le rail coulissant respectif (10, 20) pour fixer une position du support (40) par rapport à la source de rayons X (200) et au détecteur de rayons X (210).

2. Système de rail (1) selon la revendication 1, **caractérisé en ce que** le rail de guidage (30) s'étend le long d'une direction longitudinale (32) et le premier rail coulissant (10) et/ou le second rail coulissant (20) peuvent être coulissés le long de la direction longitudinale (32) par rapport au rail de guidage (30) de sorte qu'une position de la source de rayons X (200) par rapport au détecteur de rayons X (210) peut être réglée.

3. Système de rail (1) selon la revendication 2, **caractérisé en ce que** le support (40) est rotatif par rapport au premier ou au second rail coulissant (10, 20) autour d'un axe de rotation (42), dans lequel l'axe de rotation (42) s'étend de manière orthogonale à la direction longitudinale (32) du rail de guidage (30) de sorte qu'un objet (220) agencé sur le support (40) est rotatif d'un angle de rotation défini (44) d'une première position dans une seconde position ou de sorte qu'un objet (220) agencé sur le support (40) est rotatif d'une position de départ successivement dans une pluralité d'étapes de rotation d'un angle de rotation défini (44) à chaque fois de sorte qu'après un nombre défini d'étapes de rotation, l'objet (220) est de nouveau dans la position de départ, notamment dans lequel le système de rail (1) présente un moteur pas à pas (50) qui est réalisé pour tourner le support (40) autour de l'axe de rotation (42), notamment dans lequel le système de rail (1) présente un dispositif de commande (52) qui est réalisé pour commander le moteur pas à pas (50).

4. Système de rail (1) selon une des revendications 2 ou 3, **caractérisé en ce que** le système de rail (1) présente un actionneur (54) qui est réalisé pour déplacer le support (40) le long de la direction longitudinale (32).

5. Système de rail (1) selon une des revendications 2 à 4, **caractérisé en ce que**
le rail de guidage (30) présente une première face latérale (34) et une seconde face latérale (35) détournée de la première face latérale (34), dans lequel la première face latérale (34) présente une première rainure (61) et la seconde face latérale (35) une deuxième rainure (62), dans lequel la première rainure (61) et la deuxième rainure (62) s'étendent le long de la direction longitudinale (32), dans lequel
le premier rail coulissant (10) présente une première face de fond (12), dans lequel la première face de fond (12) présente une troisième rainure (63) et une quatrième rainure (64), dans lequel la troisième rainure (63) et la quatrième rainure (64) s'étendent le long de la direction longitudinale (32), dans lequel
le second rail coulissant (20) présente une seconde face de fond (22), dans lequel la seconde face de fond (22) présente une cinquième rainure (65) et une sixième rainure (66), dans lequel la cinquième rainure (65) et la sixième rainure (66) s'étendent le long de la direction longitudinale (32), dans lequel
le système de rail (1) présente au moins un premier élément porteur (70), un deuxième élément porteur (72), un troisième élément porteur (74) et un quatrième élément porteur (76), dans lequel
le système de rail (1) présente au moins un premier coulisseau (80), un deuxième coulisseau (82), un troisième coulisseau (84) et un quatrième coulisseau (86), dans lequel le premier coulisseau (80) peut être agencé dans la troisième rainure (63), le deuxième coulisseau (82) dans la quatrième rainure (64), le troisième coulisseau (84) dans la cinquième rainure (65) et le quatrième coulisseau (86) dans la sixième rainure (66), et dans lequel le premier coulisseau (80) peut être immobilisé sur le premier élément porteur (70), le deuxième coulisseau (82) sur le deuxième élément porteur (72), le troisième coulisseau (84) sur le troisième élément porteur (74) et le quatrième coulisseau (86) sur le quatrième élément porteur (76), et dans lequel
un premier élément de serrage (90) est agencé sur le premier élément porteur (70), un deuxième élément de serrage (92) sur le deuxième élément porteur (72), un troisième élément de serrage (94) sur le troisième élément porteur (74) et un quatrième élément de serrage (96) sur le quatrième élément porteur (76), dans lequel
le premier élément de serrage (90) et le troisième élément de serrage (94) peuvent être agencés dans la première rainure (61), dans lequel
le deuxième élément de serrage (92) et le quatrième élément de serrage (96) peuvent être agencés dans la deuxième rainure (62),
de sorte que le premier rail coulissant (10) et le second rail coulissant (20) peuvent être connectés au rail de guidage (30).

6. Système de rail (1) selon la revendication 5, **caractérisé en ce que** le premier élément de serrage (90) peut être calé dans la première rainure (61) de sorte que le premier élément porteur (70) peut être fixé sur le rail de guidage (30), et/ou que le deuxième élément de serrage (92) peut être calé dans la deuxième rainure (62) de sorte que le deuxième élément porteur (72) peut être fixé sur le rail de guidage (30), et/ou que le troisième élément de serrage (94) peut être calé dans la première rainure (61) de sorte que le troisième élément porteur (74) peut être fixé sur le rail de guidage (30), et/ou que le quatrième élément de serrage (96) peut être calé dans la deuxième rainure (62) de sorte que le quatrième élément porteur (76) peut être fixé sur le rail de guidage (30), et/ou que le premier élément porteur (70) présente une première face porteuse (100) et une première face d'appui (110), dans lequel la première face porteuse (100) et la première face d'appui (110) sont agencées de manière orthogonale l'une à l'autre, et/ou que le deuxième élément porteur (72) présente une deuxième face porteuse (102) et une deuxième face d'appui (112), dans lequel la deuxième face porteuse (102) et la deuxième face d'appui (112) sont agencées de manière orthogonale l'une à l'autre, et/ou que le troisième élément porteur (74) présente une troisième face porteuse (104) et une troisième face d'appui (114), dans lequel la troisième face porteuse (104) et la troisième face d'appui (114) sont agencées de manière orthogonale l'une à l'autre, et/ou que le quatrième élément porteur (76) présente une quatrième face porteuse (106) et une quatrième face d'appui (116), dans lequel la quatrième face porteuse (106) et la quatrième face d'appui (116) sont agencées de manière orthogonale l'une à l'autre, notamment dans lequel le premier élément porteur (70) peut être positionné par rapport au rail de guidage (30) et au premier rail coulissant (10) de telle sorte que la première face porteuse (100) repose sur la première face de fond (12) et la première face d'appui (110) repose sur la première face latérale (34), et/ou que le deuxième élément porteur (72) peut être positionné par rapport au rail de guidage (30) et au premier rail coulissant (10) de telle sorte que la deuxième face porteuse (102) repose sur la première face de fond (12) et la deuxième face d'appui (112) repose sur la seconde face latérale (35), et/ou que le troisième élément porteur (74) peut être positionné par rapport au rail de guidage (30) et au second rail coulissant (20) de telle sorte que la troisième face porteuse (104) repose sur la seconde face de fond (22) et la troisième face d'appui (114) repose sur la première face latérale (34), et/ou que le quatrième élément porteur (76) peut être positionné par rapport au rail de guidage (30) et au second rail coulissant (20) de telle sorte que la quatrième face porteuse (106) repose sur la seconde face de fond (22) et la quatrième face d'appui (116) repose sur la seconde face latérale (35).

7. Système de rail (1) selon une des revendications 5 à 6, **caractérisé en ce que** le système de rail (1) présente un premier moyen de fixation qui est réalisé pour immobiliser le premier coulisseau (80) dans la troisième rainure (63), et/ou présente un deuxième moyen de fixation qui est réalisé pour immobiliser le deuxième coulisseau (82) dans la quatrième rainure (64), et/ou présente un troisième moyen de fixation qui est réalisé pour immobiliser le troisième coulisseau (84) dans la cinquième rainure (65), et/ou présente un quatrième moyen de fixation qui est réalisé pour immobiliser le quatrième coulisseau (86) dans la sixième rainure (66), et/ou que le rail de guidage (30), le premier rail coulissant (10) et le second rail coulissant (20) sont réalisés pour être agencés l'un par rapport à l'autre de telle sorte que la première et la seconde face latérale (34, 35) s'étendent perpendiculairement à la première face de fond (12), et la première et la seconde face latérale (34, 35) s'étendent perpendiculairement à la seconde face de fond (22).

8. Système de rail (1) selon une des revendications 2 à 7, **caractérisé en ce que** le support (40) présente un coulisseau de support et le premier rail coulissant (10) présente une première face de recouvrement (14), dans lequel la première face de recouvrement (14) présente une première rainure de support (15), dans lequel la première rainure de support (15) s'étend le long de la direction longitudinale (32), et dans lequel le coulisseau de support est réalisé pour être agencé dans la première rainure de support (15), et/ou que le second rail coulissant (20) présente une seconde face de recouvrement (24), dans lequel la seconde face de recouvrement (24) présente une seconde rainure de support (25), dans lequel la seconde rainure de support (25) s'étend le long de la direction longitudinale (32), et dans lequel le coulisseau de support est réalisé pour être agencé dans la seconde rainure de support (25), et/ou que le support (40) présente une charnière de sorte que le support (40) peut être basculé autour d'un axe de basculement (142), dans lequel l'axe de basculement (142) s'étend de manière orthogonale à la direction longitudinale (32) du rail de guidage (30).

9. Système de rail (1) selon une des revendications précédentes, **caractérisé en ce que** le système de rail (1) présente une pluralité d'éléments de soutien (150), dans lequel l'élément de soutien respectif (150) présente une pièce angulaire (152), une tige de connexion (154) pouvant être agencée sur la pièce angulaire (152) et un pied (156) agencé sur la tige de connexion (154), dans lequel la pièce angulaire (152) est réalisée pour être immobilisée sur le rail de guidage (30), et dans lequel un écart entre la pièce angulaire (152) et le pied (156) peut être réglé à l'aide de la tige de connexion (154).

10. Système de rail (1) selon une des revendications précédentes, **caractérisé en ce que** le premier rail coulissant (10), le second rail coulissant (20) et le support (40) peuvent être immobilisés de manière amovible sur le rail de guidage (30) de sorte que le système de rail (1) peut être décomposé en le premier rail coulissant (10), le second rail coulissant (20), le support (40) et le rail de guidage (30).

11. Système de rail (1) selon une des revendications précédentes, **caractérisé en ce que** le système de rail (1) présente un dispositif d'analyse (160), dans lequel le dispositif d'analyse (160) est réalisé pour le traitement, l'évaluation et/ou la sauvegarde d'un ensemble de données, notamment dans lequel le dispositif d'analyse (160) est réalisé pour effectuer un algorithme de transformation qui crée au moins une radiographie en coupe de l'objet (220) à l'aide d'une pluralité de radiographies de projection.

12. Dispositif d'imagerie (3) pour la génération de radiographies de projection d'un objet (220), dans lequel le dispositif d'imagerie (3) présente un système de rail (1) selon une des revendications précédentes, ainsi qu'en outre une source de rayons X (200) et un détecteur de rayons X (210), dans lequel le premier rail coulissant (10) est réalisé pour porter la source de rayons X (200), et dans lequel le second rail coulissant (20) est réalisé pour porter le détecteur de rayons X (210).

13. Dispositif d'imagerie (3) selon la revendication 12, **caractérisé en ce que** le détecteur de rayons X (210) présente une plaque numérique de détecteur de rayons X, et/ou que la source de rayons X (200) est une source de rayons X portable, et/ou que le dispositif d'imagerie présente un dispositif d'analyse (160), dans lequel le dispositif d'analyse (160) est réalisé pour effectuer un algorithme de transformation qui crée au moins une radiographie en coupe de l'objet (220) à l'aide d'une pluralité de radiographies de projection.

14. Procédé de génération d'une radiographie de projection d'un objet (220) par l'utilisation d'un système de rail (1) selon une des revendications 1 à 11 ou par l'utilisation d'un dispositif d'imagerie (3) selon une des revendications 12 à 13, dans lequel un objet (220) est agencé sur le support (40) du système de rail (1) et au moins une radiographie de projection de l'objet (220) est générée au moyen de la source de rayons X (200) immobilisée sur le premier rail coulissant (10) et du détecteur de rayons X (210) immobilisé sur le second rail coulissant (20).

15. Procédé selon la revendication 14, **caractérisé en ce que** l'objet (220) agencé sur le support (40) est tourné d'une première position dans une seconde position au moyen du système de rail (1), et une première radiographie de projection de l'objet (220) est générée dans la première position et une seconde radiographie de projection de l'objet (220) dans la seconde position, notamment dans lequel au moins une radiographie en coupe de l'objet (220) est créée à partir d'une pluralité de radiographies de projection.
